# EUROPÄISCHE PATENTSCHRIFT

(11) **EP 3 565 449 B1**
(45) Veröffentlichungstag und Bekanntmachung des Hinweises auf die Patenterteilung: **07.10.2020**
(21) Anmeldenummer: 18725165.7
(22) Anmeldetag: 14.05.2018
(51) Int. Cl.: A61B 1/00, A61B 34/30, H01R 13/00, H01R 12/70, H01R 13/62

(54) **MEDIZINISCHE MECHATRONISCHE MÄNNLICHE SOWIE WEIBLICHE SCHNITTSTELLENEINRICHTUNG**
MEDICAL MECHATRONIC MALE AND FEMALE INTERFACE DEVICE
DISPOSITIF MÉDICAL MÉCATRONIQUE D'INTERFACE MÂLE ET FEMELLE

(30) Priorität: 23.05.2017 DE 102017111302
(43) Veröffentlichungstag der Anmeldung: 13.11.2019
(73) Patentinhaber: medineering GmbH, 81829 München (DE)
(72) Erfinder: KRINNINGER, Maximilian, 82234 Wessling-Oberpfaffenhofen (DE); NOWATSCHIN, Stephan, 81677 München (DE); KÜHNAU, Christian, 81829 München (DE); ROPPENECKER, Daniel, 81829 München (DE); GIERLACH, Dominik, 80798 München (DE); AGRICOLA, Johannes, 81829 München (DE); HOFBERGER, Stefan, 81829 München (DE)
(74) Vertreter: Eisenführ Speiser
(86) Internationale Anmeldenummer: PCT/EP2018/062327
(87) Internationale Veröffentlichungsnummer: WO 2018/215223

(56) Entgegenhaltungen:
- EP-A1- 3 130 305
- EP-A1- 3 130 305
- WO-A1-2016/119001
- WO-A1-2016/119001
- CA-A1- 2 527 536
- DE-A1- 19 526 915
- DE-A1-102011 003 036
- US-A1- 2017 095 300

## Beschreibung

Die Erfindung betrifft eine medizinische mechatronische männliche Schnittstelleneinrichtung zum Koppeln eines chirurgischen Assistenzsystems an eine Haltestruktur, mit einer Basis, die eine Stirnseite aufweist, wobei sich von der Stirnseite ein Funktionskörper erstreckt. Die Erfindung betrifft ferner eine korrespondierende medizinische weibliche Schnittstelleneinrichtung zum Koppeln mit der männlichen Schnittstelleneinrichtung. Die Erfindung betrifft auch einen Haltearm für medizinische Zwecke zum Halten eines chirurgischen Assistenzsystems mit einem proximalen Ende zum Befestigen des Haltearms an einer Basis und einem distalen Ende zum Aufnehmen des chirurgischen Assistenzsystems, zwei oder mehr Armsegmenten und zwei oder mehr Gelenken, mittels denen die Armsegmente gelenkig miteinander verbunden sind; einer ersten Schnittstelle an dem proximalen Ende zum Verbinden des Haltearms mit eine Energiequelle sowie mit einer externen Steuereinheit zum Übertragen von Signalen an den und von dem Haltearm; einer zweiten Schnittstelle an dem distalen Ende zum Koppeln des Haltearms mit einem Assistenzsystem zum Steuern des Assistenzsystems; und einer Übertragungseinrichtung, welche innerhalb des Haltearms angeordnet ist und die erste Schnittstelle mit der zweiten Schnittstelle zum Übertragen von Energie und Signalen zwischen den Schnittstellen verbindet. Ferner betrifft die Erfindung ein chirurgisches Assistenzsystem mit einer medizinischen weiblichen Schnittstelleneinrichtung sowie ein System mit einem Haltearm der vorstehend genannten Art und einem oder mehreren chirurgischen Assistenzsystemen der vorstehend genannten Art.

Haltearme der eingangs genannten Art sind bereits seit längerem bekannt und werden in der Chirurgie insbesondere dazu eingesetzt, einen Operateur von statischer Haltearbeit zu entlasten. Ein derartiger Haltearm wird eingesetzt, um ein chirurgisches Assistenzsystem zu halten, wie etwa einen Manipulator, ein Endoskop, eine Klemme und dergleichen. Insbesondere zum Halten von Endoskopen haben sich die eingangs genannten Haltearme bewährt. Bei der endoskopischen Chirurgie bedient ein Operateur in der Regel mit beiden Händen ein Instrument, während ein Assistent das Endoskop hält, um das Operationsfeld über einen Bildschirm sichtbar zu machen. Das Halten des Endoskops über einen längeren Zeitraum, ist sehr ermüdend. Aus diesem Grund werden vermehrt Haltearme eingesetzt.

Eine solcher Haltearm ist beispielsweise aus DE 195 26 915 B4 bekannt. Die dort offenbarte Haltevorrichtung für medizinische Zwecke weist ein Anschlussteil und einen Halter für chirurgische Werkzeuge sowie einen zwischen dem Halter und dem Anschlussteil angeordneten Arm auf. Der Arm ist mit dem Halter und dem Anschlussteil oder mit einem benachbarten Arm über ein Gelenk verbunden und mit einer pneumatisch betätigbaren Vorrichtung zur wahlweisen Festlegung und Lösung der Gelenke gekoppelt, wobei diese Vorrichtung die Gelenke unter Einwirkung einer eine Bremskraft auf das Gelenk ausübenden mechanischen Feder festlegt und wobei die Vorrichtung gegen die Kraft dieser Feder pneumatisch in einen das Gelenk freigebenden Zustand überführbar ist. An dem Halter am proximalen Ende des Arms ist ein Betätigungsorgan angeordnet, mittels dessen Hilfe ein Ventil öffenbar ist, sodass die einzelnen Gelenke des Arms verstellt werden können. Bei Loslassen des Betätigungsorgans wird das Ventil wieder geschlossen, sodass die Gelenke festgelegt sind.

Ein ähnlicher Haltearm ist in EP 1 958 587 B1 offenbart. Der dort offenbarte Haltearm weist ebenfalls mehrere Gelenke auf, und zur Betätigung der Gelenke ist ein berührungssensitiver Sensor vorgesehen. Dieser Sensor ist am Haltearm benachbart zum medizinischen Instrument angeordnet, sodass bei Ergreifen des medizinischen Instruments der Operator in Kontakt mit dem berührungssensitiven Sensor kommt, wodurch die Gelenke des Haltearms freigegeben werden.

Beide oben genannten Haltearme sind sogenannte passive Haltearme, die ausschließlich gebremste Gelenke aufweisen, jedoch keine aktiven Antriebe in den Gelenken. Die Gelenke sind aufgrund der Betätigung der Bedieneinrichtung freigebbar und bei Loslassen dieser wieder arretiert.

Ein weiterer derartiger Haltearm ist in DE 10 2011 004 926 A1 offenbart. Der Haltearm weist mehrere Armsegmente sowie mehrere Gelenke auf, mittels derer die einzelnen Armsegmente miteinander gekoppelt sind. Der Haltearm gemäß DE 10 2011 004 926 A1 weist ferner eine erste Schnittstelle an dem proximalen Ende auf, um den Haltearm mit einer Normschiene eines Operationstisches zu koppeln. Die erste Schnittstelle ist im Wesentlichen gemäß einer Klemme ausgebildet. Darüber hinaus weist der Haltearm eine Schnittstelle am distalen Ende auf, die ebenfalls als Klemme ausgebildet ist und dazu dient, ein Endoskop aufzunehmen. Auch wenn sich dieser Arm grundsätzlich gut zum reinen Halten von Endoskopen eignet, besteht dennoch ein Bedarf, den Einsatzbereich solcher Haltearme flexibler zu gestalten, insbesondere an verschiedene Aufgaben anzupassen. Ferner ist es wünschenswert, die Sicherheit derartiger Haltearme dahingehend zu verbessern, dass ein Verletzungsrisiko eines Patienten während einer Operation, bei der der Haltearm eingesetzt wird, verringert wird.

Ein Haltearm mit den Merkmalen des Oberbegriffs des Anspruchs 1 ist weiterhin aus DE 10 2014 016 823 der hiesigen Anmelderin bekannt. Ein weiterer Haltearm der hiesigen Anmelderin ist aus EP 3 130 305 A1 bekannt.

Bei solchen Haltearmen besteht der Bedarf, verschiedene Assistenzsysteme anschließen zu können. Insbesondere ist abhängig von der Art der Operation bzw. der Art der Halteaufgabe erforderlich, verschiedene solche Systeme anschließen zu können. Darüber hinaus haben verschiedene Hersteller verschiedene Schnittstellen, und es ist bevorzugt, dass diese verschiedenen Assistenzsysteme, wie insbesondere Instrumente und dergleichen, an dem Haltearm anschließbar sind.

Ferner ist aus WO 2016/119001 A1 eine Steckverbindung zum Verbinden von insbesondere elektrischen Leitungen bekannt, umfassend zumindest ein weibliches Verbindungselement und ein männliches Verbindungselement, wobei das weibliche Verbindungselement das männliche Verbindungselement formschlüssig aufnimmt und wobei die beiden Verbindungselemente bei formschlüssigem Kontakt kraftschlüssig lösbar verbindbar sind. Ein in Verbindung tretender Bereich des männlichen Verbindungselementes ist koaxial zulaufend ausgebildet und umfasst zumindest einen verschiebbaren Kontaktkörper, welcher in einer ersten Position innerhalb und in einer zweiten Position aus dem männlichen Verbindungselement vorragend angeordnet ist.

Aus US 2017 0095300 A1 sind eine männliche und weibliche Schnittstelle eines medizinischen Geräts bekannt. Die männliche Schnittstelle weist zentral eine flächige Erhebung auf, die in eine entsprechende Vertiefung der weiblichen Schnittstelle eingreifen kann. Zudem sind elektrische Kontakte und Arretierungen vorgesehen.

Aufgabe der vorliegenden Erfindung ist es, sowohl eine medizinische mechatronische männliche Schnittstelleneinrichtung, also auch eine medizinische weibliche Schnittstelleneinrichtung der eingangs genannten Art bereitzustellen, durch die ein chirurgisches Assistenzsystem auf einfache Art und Weise anschließbar ist und weiterhin die Einsatzmöglichkeiten vergrößert sind.

Die Erfindung löst die Aufgabe bei einer medizinischen mechatronischen männlichen Schnittstelleneinrichtung dadurch, dass diese erste mechanische Kopplungsmittel zum form- und/oder kraftschlüssigen Koppeln mit einer korrespondierenden medizinischen mechatronischen weiblichen Schnittstelleneinrichtung sowie wenigstens eine erste elektronische Schnittstelle zum Verbinden mit einer korrespondierenden zweiten elektronischen Schnittstelle der weiblichen Schnittstelleneinrichtung aufweist, wobei der Funktionskörper eine Zentriereinrichtung zum spielfreien Zentrieren des chirurgischen Assistenzsystems gegenüber der Basis aufweist, wobei die Zentriereinrichtung eine erste Funktionsfläche eine zweite Funktionsfläche und eine dritte Funktionsfläche umfasst, die nicht parallel zueinander sind und deren Normalen nicht in einer gemeinsamen Ebene liegen.

Die Erfindung macht sich zunutze, dass eine Schnittstelleneinrichtung nicht nur mechanische Kopplungsmittel zum form- und/oder kraftschlüssigen Koppeln aufweisen muss, um eine Verbindung herzustellen, sondern dass insbesondere eine Zentriereinrichtung bevorzugt ist, sodass die korrespondierende medizinische mechatronische weibliche Schnittstelleneinrichtung mit der männlichen Schnittstelleneinrichtung zentriert gekoppelt werden kann. Durch eine Zentrierung ist es möglich, leichte Fertigungsungenauigkeiten auszugleichen und eine spielfreie Kupplung zu erzeugen.

Indem die Funktionsflächen nicht parallel zueinander sind und ihre Normalen nicht in einer gemeinsamen Ebene liegen, ist es möglich, eine statisch bestimmte Kupplung mit der korrespondierenden weiblichen Schnittstelleneinrichtung zu erzeugen. Die korrespondierende weibliche Schnittstelleneinrichtung weist vorzugsweise entsprechende Funktionsflächen, oder andere Kupplungselemente auf, die mit den ersten, zweiten, dritten Funktionsflächen der männlichen Schnittstelleneinrichtung in Kontakt kommen.

Die Haltestruktur kann im Rahmen der Erfindung jegliche Haltestruktur sein, an der ein Bedarf zur Befestigung eines chirurgischen Assistenzsystem bestehen kann, wie zum Beispiel ein Stativ, ein Deckenstativ, ein OP-Tisch, eine Raumwand, ein Haltearm, ein Roboter, und ähnliches.

Auch wenn es bevorzugt ist, dass die männliche Schnittstelleneinrichtung eine elektronische Schnittstelle aufweist, sind auch Ausführungsformen ohne die elektronische Schnittstelle bevorzugt. In diesem Fall ist die männliche Schnittstelleneinrichtung rein mechanisch ausgebildet.

Die ersten zweiten und dritten Funktionsflächen definieren erfindungsgemäß gemeinsam wenigstens teilweise eine dreiseitige Pyramide. Durch die Anordnung der Normalen der Funktionsflächen, das heißt der Normalen, die senkrecht auf Ebenen stehen, die die Funktionsflächen definieren, ist eine pyramidale Struktur erreicht, die vorzugsweise von der Basis weg spitz zuläuft.

Bevorzugt ist auch der Funktionskörper in sich spiegelsymmetrisch.

Bevorzugt ist der Funktionskörper in sich spiegelsymmetrisch. Dabei ist es in einer Ausführungsform bevorzugt, dass die Pyramide spiegelsymmetrisch ist. Die Symmetrieebene umfasst vorzugsweise die Zentralachse der pyramidalen Struktur bzw. die Zentralachse des Funktionskörpers und ist vorzugsweise senkrecht auf die Basis. Hierdurch kann die Konstruktion weiter vereinfacht werden und es ist möglich, Gleichteile zu verwenden.

Weiter wird bevorzugt, dass die Funktionsflächen eben sind. Es reicht in diesem Sinne aus, wenn die Funktionsflächen im Wesentlichen eben sind, und eine leichte Balligkeit oder Konkavität wird hingenommen. So kann es beispielsweise denkbar sein, dass aus Gründen einer Verspannung der männlichen und der weiblichen Schnittstelleneinrichtung zueinander eine leicht regelmäßig oder unregelmäßig gekrümmte Fläche bevorzugt ist. Allerdings ist es möglich, durch das Vorsehen von ebenen Funktionsflächen die Fertigung zu vereinfachen, und auch eine statische Bestimmtheit bei einer Kopplung mit einer korrespondierenden weiblichen Schnittstelleneinrichtung kann auf diese Weise besser erreicht werden.

Besonders bevorzugt weisen die Funktionsflächen jeweils einen Winkel von 60° zueinander auf. Hierdurch ist eine Symmetrie der Funktionsflächen erreicht. In einer weiter bevorzugten Variante sind zwei Funktionsflächen identisch zueinander ausgebildet, aber spiegelsymmetrisch, während die dritte Funktionsfläche im Wesentlichen senkrecht zur Symmetrieebene ist.

Insofern ist es auch möglich, dass zwei Funktionsflächen einen geringeren Winkel als 60° zueinander einschließen, beispielsweise 45°, und diese beiden Funktionsflächen dann mit der dritten Funktionsfläche jeweils einen größeren Winkel, nämlich vorzugsweise 67,5° einschließen.

Besonders bevorzugt ist, dass die Funktionsflächen jeweils wenigstens 20° zu einer Zentralachse des Funktionskörpers geneigt sind. Das heißt, zur Zentralachse der pyramidalen Struktur sind die Funktionsflächen um jeweils wenigstens 20° geneigt. Eine geringere Neigung, d.h. eine Neigung nahe von 20° und vorzugsweise kleiner als 30° kann zu einer besseren Positionierung senkrecht zur Zentralachse führen. Größere Neigungswinkel können hingegen zu einer besseren Positionierung in axialer Richtung führen. Bei Winkeln, die kleiner als 20° sind, besteht die Gefahr, dass Selbsthemmung eintritt und insofern ein Lösen der Kopplung der männlichen Schnittstelleneinrichtung mit der weiblichen Schnittstelleneinrichtung nicht mehr ohne Weiteres möglich ist.

Gemäß einer weiter bevorzugten Ausführungsform ist vorgesehen, dass der Funktionskörper eine axiale Stirnseite aufweist. Insofern ist vorgesehen, dass die Pyramide, bzw. pyramidale Struktur einem Pyramidenstumpf ähnelt und nicht in einer axialen Spitze mündet. Eine Axiale Spitze ist nicht erforderlich, um eine Kopplung zwischen der männlichen Schnittstelleneinrichtung und der weiblichen Schnittstelleneinrichtung zu erzeugen. In dieser Ausführungsform ist daher die Pyramide, bzw. pyramidale Struktur, zu einem Stumpf gekürzt und der Funktionskörper weist eine axiale Stirnseite auf. Die axiale Stirnseite ist vorzugsweise parallel zur Stirnseite der Basis und/oder senkrecht zur Zentralache der Pyramide. In anderen Ausführungsformen ist es denkbar, dass die Stirnseite des Funktionskörpers geneigt zur Zentralachse angeordnet ist.

Gemäß einer weiter bevorzugten Ausführungsform weisen die ersten mechanischen Kopplungsmittel wenigstens eine erste Hinterschneidung an dem Funktionskörper auf. Die Hinterschneidung ist vorzugsweise durch eine Ausnehmung an dem Funktionskörper ausgebildet und definiert einen hintergreifbaren Abschnitt. Vorzugsweise ist eine zweite Hinterschneidung an dem Funktionskörper ausgebildet, die besonders bevorzugt spiegelsymmetrisch zur ersten Hinterschneidung ausgebildet ist.

Vorzugsweise sind die ersten und zweiten Hinterschneidungen radial zugänglich, sodass ein entsprechendes Sperrglied von zweiten mechanischen Kopplungsmitteln in diese Hinterschneidung eingreifen kann.

In einer weiteren bevorzugten Ausführungsform weisen die ersten mechanischen Kopplungsmittel eine dritte Hinterschneidung an dem Funktionskörper auf. Mit insgesamt drei Hinterschneidungen kann eine besonders feste Sicherung der männlichen und der weiblichen Schnittstelleneinrichtung aneinander erreicht werden. Es soll verstanden werden, dass es auch Ausführungsformen geben kann, bei denen die ersten mechanischen Kopplungsmittel nur die dritte Hinterschneidung, nicht aber die ersten und zweiten Hinterschneidungen aufweisen.

Vorzugsweise ist dabei die dritte Hinterschneidung im Wesentlichen entgegengesetzt zu der ersten und/oder zweiten Hinterschneidung ausgebildet, sodass ein entsprechender Vorsprung in einer mit Bezug auf die erste und/oder zweite Hinterschneidung entgegengesetzte Richtung in die dritte Hinterschneidung eingreifen kann. Wenn also beispielsweise Vorsprünge für die ersten und zweiten Hinterschneidungen von oben oder schräg oben in diese eingreifen, greift ein für die dritte Hinterschneidung vorgesehener Vorsprung von unten in diese ein. Durch entgegengesetzte Richtungen kann einem ungewollten Lösen besser entgegengewirkt werden.

Weiterhin ist bevorzugt, dass die dritte Hinterschneidung bezogen auf eine Mittelebene, vorzugsweise Symmetrieebene, des Funktionskörpers im Wesentlichen mittig zwischen der ersten und zweiten Hinterschneidung angeordnet ist. Hierdurch kann eine gleichmäßigere Verteilung von Kräften erreicht werden.

In einer weiteren bevorzugten Ausführungsform laufen die Funktionsflächen radial und/oder umfänglich in eine Umfangsfläche eines Grundkörpers des Funktionskörpers aus.

Vorzugsweise ist zwischen den Funktionsflächen und der Umfangsfläche des Grundkörpers des Funktionskörpers ein glatter Übergang vorgesehen, der knickfrei ist. Hierdurch kann die Reinigung der Schnittstelleneinrichtung vereinfacht werden und die Hygiene verbessert werden. Ein Radius in dem Übergangsbereich zwischen den Funktionsflächen und der Umfangsfläche ist vorzugsweise in einem Bereich von 1mm und mehr, vorzugsweise 0,5mm und mehr.

Der Grundkörper des Funktionskörpers kann grundsätzlich jede Form haben, aber ist vorzugsweise nicht rotationssymmetrisch. Hierdurch ist es möglich, dass auch der Grundkörper einen Teil der mechanischen Kopplungsmittel bilden kann und insbesondere in einer Rotation der weiblichen Schnittstelleneinrichtung gegenüber der männlichen Schnittstelleneinrichtung entgegenwirken. Vorzugsweise ist der Grundkörper im Wesentlichen zylindrisch mit einer radialen Nase ausgebildet. Der so ausgebildete Grundkörper erstreckt sich vorzugsweise axial von der Basis. Ein erster axialer Abschnitt des Funktionskörpers ist dabei vorzugsweise frei von Funktionsflächen; die Funktionsflächen sind vorzugsweise nur in einem zweiten axialen Abschnitt des Grundkörpers vorgesehen, der distal von der Basis ist. Vorzugsweise ist die axiale Länge des zweiten Abschnitts in etwa gleich zur axialen Länge des ersten Abschnitts.

Besonders bevorzugt ist der Funktionskörper insgesamt einstückig ausgebildet. beispielsweise ist der Funktionskörper aus einem Vollmaterial gefräst, oder durch urformende Verfahren hergestellt. Hierdurch wird vermieden, dass der Funktionskörper Fügestellen oder dergleichen aufweist und die Hygiene kann verbessert werden. Für den Fall, dass in dem Funktionskörper Durchbrüche oder dergleichen vorgesehen sind, beispielsweise für Schrauben oder Anschlüsse elektrischer pneumatischer oder hydraulischer Art, oder Anschlüsse für das Durchleiten von Funktionsflüssigkeiten, wie insbesondere therapeutischen Flüssigkeiten, Medikamenten und dergleichen, sind diese Öffnungen oder Anschlüsse vorzugsweise abgedichtet. Hierdurch soll vermieden werden, dass Flüssigkeit von außerhalb des Funktionskörpers in das Innere des Funktionskörpers eindringen kann.

In einer weiteren bevorzugten Ausführungsform, ist die elektronische Schnittstelle an der Stirnseite der Basis ausgebildet. Die weibliche Schnittstelleneinrichtung wird vorzugsweise in axialer Richtung bezogen auf den Funktionskörper und auch in axialer Richtung bezogen auf die Basis mit der männlichen Schnittstelleneinrichtung gekoppelt. Indem die elektronische Schnittstelle an der Stirnseite der Basis ausgebildet ist, steht einerseits der Funktionskörper vollständig zur mechanischen Kopplung und auch zum Übertragen von Fluiden oder dergleichen zur Verfügung. Andererseits ist es möglich, dass die elektronische Schnittstelle mit einer übereinstimmenden Kopplungsrichtung, wie der Funktionskörper ausgestattet ist. Das heißt, auch die elektronische Schnittstelle wird vorzugsweise in axialer Richtung gekoppelt. Die elektronische Schnittstelle umfasst vorzugsweise eine Steckverbindung.

Vorzugsweise umfasst die elektronische Schnittstelle zwei oder mehr Blöcke mit elektronischen Kontakten. Jeder Block weist vorzugsweise eine Mehrzahl, zwei oder mehr elektronische Kontakte auf. Die Blöcke können redundant ausgebildet sein, das heißt, dass zwei oder mehr Blöcke dieselben elektronischen Kontakte aufweisen und insofern als redundante Blöcke verwendet werden können. Die Blöcke können aber auch in anderen Ausführungsformen unterschiedlich belegt sein. Durch das Vorsehen von mehreren Blöcken, ist es möglich diese mehreren Blöcke gegenseitig besser abzuschirmen. Die elektrischen Kontakte ihrerseits sind vorzugsweise isoliert und abgedichtet gegen Flüssigkeiten.

Weiterhin ist bevorzugt, dass die elektronische Schnittstelle wenigstens zwei Erdungskontakte aufweist, die derart angeordnet sind, dass wenigstens einer von diesen bei Kopplung mit einer korrespondierenden weiblichen Schnittstelleneinrichtung zuerst mit wenigstens einem korrespondierenden Erdungskontakt der weiblichen Schnittstelleneinrichtung in Kontakt kommt, bevor weitere elektrische Kontakte geschlossen werden. Hierdurch ist die elektronische Schnittstelle geerdet, bevor die übrigen Kontakte geschlossen werden. Auf diese Weise ist es möglich und denkbar, dass die Schnittstelleneinrichtungen bei anliegender Spannung gekoppelt und entkoppelt werden, also ein sogenanntes "Hot-Plug". Dies wird beispielsweise dadurch realisiert, dass die Erdungskontakte in axialer Richtung weiter vorstehen, um so die zeitliche Abfolge beim Koppeln der Schnittstelleneinrichtungen zu erreichen.

Gemäß einem zweiten Aspekt der Erfindung wird die eingangs genannte Aufgabe gelöst durch eine medizinische weibliche Schnittstelleneinrichtung zum Koppeln eines chirurgischen Assistenzsystems an eine Haltestruktur, wobei die Schnittstelleneinrichtung zum Koppeln mit der männlichen Schnittstelleneinrichtung nach einer der vorstehend beschriebenen bevorzugten Ausführungsformen der medizinischen mechatronischen männlichen Schnittstelleneinrichtung ausgebildet ist, mit: einem Rahmen, der eine zentrale Ausnehmung zur Aufnahme des Funktionskörpers definiert, zweiten mechanischen Kopplungsmitteln zum form- und/oder kraftschlüssigen Koppeln mit den ersten mechanischen Kopplungsmitteln der männlichen Schnittstelleneinrichtung, wobei die zentrale Ausnehmung eine Zentriereinrichtung zum spielfreien Zentrieren des chirurgischen Assistenzsystems gegenüber der Basis der ersten Schnittstelleneinrichtung aufweist, wobei die Zentriereinrichtung eine erste Funktionsfläche, eine zweite Funktionsfläche und eine dritte Funktionsfläche umfasst, die mit den ersten, zweiten und dritten Funktionsflächen der männlichen Schnittstelleneinrichtung zum flächigen Kontaktieren korrespondieren. Es soll verstanden werden, dass die männliche Schnittstelleneinrichtung gemäß dem ersten Aspekt der Erfindung und die weibliche Schnittstelleneinrichtung gemäß dem zweiten Aspekt der Erfindung gleiche und ähnliche Unteraspekte aufweisen, wie sie insbesondere in den abhängigen Ansprüchen niedergelegt sind. Insofern wird vollumfänglich auf die obige Beschreibung bezüglich der weiteren bevorzugten Merkmale, deren Vorteile sowie Kombinations- und Einsatzmöglichkeiten Bezug genommen. Die ersten, zweiten, dritten Funktionsflächen der weiblichen Schnittstelleneinrichtung müssen nicht zwangsläufig identisch zu den ersten, zweiten und dritten Funktionsflächen der männlichen Schnittstelleneinrichtung ausgebildet sein. So ist es beispielsweise denkbar, dass die ersten, zweiten und dritten Funktionsflächen der weiblichen Schnittstelleneinrichtung kleiner in ihrer flächigen Ausdehnung sind, oder eine andere Kontur aufweisen. Beispielsweise ist es denkbar, dass die Funktionsflächen der weiblichen Schnittstelleneinrichtung insgesamt ballig oder konvex ausgebildet sind, beispielsweise durch drei sphärische Körper gebildet sind.

Die zweiten mechanischen Kopplungsmittel weisen vorzugsweise wenigstens einen ersten beweglichen Bolzen zum Hintergreifen der ersten Hinterschneidung der ersten mechanischen Kopplungsmittel auf. Vorzugsweise weisen die zweiten mechanischen Kopplungsmittel auch einen zweiten Bolzen auf. Der erste und/oder zweite Bolzen ragt vorzugsweise im Wesentlichen radial in die Ausnehmung hinein, die die Hinterschneidung bildet.

Vorzugsweise sind der erste und bevorzugt auch der zweite Bolzen in eine Arretierstellung vorgespannt. Der oder die Bolzen sind vorzugsweise zwischen einer Arretierstellung und einer Freigabestellung bewegbar, wobei sie in der Arretierstellung in die Hinterschneidung, bzw. Hinterschneidungen der ersten mechanischen Kopplungsmittel eingreifen. Die Vorspannung in die Arretierstellung wird vorzugsweise mittels einer Feder, beispielsweise einer Spiralfeder, einer Drehfeder, oder auf pneumatische Weise gelöst. Auch ist es denkbar eine magnetische Vorspannung einzusetzen, um den oder die Bolzen in die Arretierstellung vorzuspannen.

Besonders bevorzugt ist der erste Bolzen an der freien Spitze angeschrägt. Hierdurch können einerseits Kupplungstoleranzen ausgeglichen werden. Dies gilt vorzugsweise auch für den zweiten Bolzen. Die angeschrägte Spitze dient vorzugsweise dazu, in Kontakt mit einer Oberfläche der Hinterschneidung zu kommen und durch die Anschrägung kann einerseits erreicht werden, dass Kupplungstoleranzen ausgeglichen werden, aber auch, dass die weibliche Schnittstelleneinrichtung beim Verbringen des Bolzens in die Arretierstellung gegen die erste Schnittstelleneinrichtung aufgrund einer Keilwirkung der schrägen Fläche des Bolzens gezogen wird.

In einer bevorzugten Ausgestaltung weisen die zweiten mechanischen Kopplungsmittel ferner einen dritten beweglichen Bolzen zum Hintergreifen der dritten Hinterschneidung der ersten mechanischen Kopplungsmittel auf. Sofern die ersten mechanischen Kopplungsmittel nur die dritte Hinterschneidung aufweisen, weisen die zweiten mechanischen Kopplungsmittel vorzugsweise nur den dritten Bolzen auf.

Durch eine Kombination von drei Bolzen ist es möglich einem Rotieren um eine Ache quer zur Zentralachse besonders effektiv entgegenzuwirken.

Vorzugsweise ist der dritte Bolzen federbelastet vorgespannt und wird bei einem Verbinden der weiblichen Schnittstelleneinrichtung mit der männlichen Schnittstelleneinrichtung selbsttätig ausgelenkt und rastet in die Hinterschneidung ein, um einem Lösen entgegenzuwirken. Der dritte Bolzen ist vorzugsweise als Rastbolzen ausgebildet. Ein Lösen des dritten Bolzens ist vorzugsweise nur möglich, wenn zunächst die ersten und/oder zweiten Bolzen gelöst werden.

Weiterhin ist vorzugsweise eine Betätigungseinrichtung vorgesehen, durch die die zweiten mechanischen Kopplungsmittel in eine Freigabestellung versetzt werden können. Diese Betätigungseinrichtung umfasst vorzugsweise einen schwenkbar am Rahmen gelagerten Hebel mit einem Griffabschnitt, der abtriebsseitig mit dem ersten Bolzen in Kontakt steht, wobei durch Betätigung des Griffabschnitts der erste Bolzen von der Arretierstellung in die Freigabestellung bringbar ist. Die Betätigungseinrichtung ist in diesem Beispiel manuell betätigbar. Es kann auch vorgesehen sein, dass eine elektronisch und/oder pneumatisch aktivierbare Betätigungseinrichtung vorgesehen ist. Dies ist besonders dann bevorzugt, wenn die Arretiereinrichtung magnetisch, insbesondere elektromagnetisch wirkt. Bevorzugt wird die Betätigung durch Drücken eines Tasters oder eines Knopfs aktiviert.

In einer Variante wird auch der dritte Bolzen mittels der Betätigungseinrichtung betätigt. In einer anderen Variante ist eine zweite Betätigungseinrichtung zum Versetzen des dritten Bolzens in eine Freigabestellung unabhängig vom ersten Bolzen vorgesehen. Hierdurch kann die Sicherheit verbessert werden.

In einer weiteren bevorzugten Ausgestaltung ist der erste Bolzen über wenigstens ein Filmscharnier mit der Betätigungseinrichtung gekoppelt. Der Bolzen ist in diesem Ausführungsbeispiel vorzugsweise einstückig mit der Betätigungseinrichtung verbunden, insbesondere nicht reversibel lösbar und nicht zerstörungsfrei lösbar. Ein Filmscharnier hat den Vorteil, dass es spielfrei arbeitet und gleichzeitig eine Dichtung bilden kann. Ferner ist ein Filmscharnier insbesondere bei einer Fertigung der männlichen und/oder weiblichen Schnittstelleneinrichtungen wenigstens teilweise aus Kunststoff bevorzugt.

Weiterhin ist bevorzugt, dass die zweite elektronische Schnittstelle federnd gelagert ist. Hierdurch können wiederum Kupplungstoleranzen ausgeglichen werden. Es soll aber verstanden werden, dass auch möglich ist, die erste elektronische Schnittstelle federnd zu lagern.

Weiter oben wurde mit Bezug auf die männlichen und weiblichen Schnittstelleneinrichtungen beschrieben, dass die männliche Schnittstelleneinrichtung die ersten mechanischen Kopplungsmittel aufweist und die weibliche Schnittstelleneinrichtung die zweiten mechanischen Kopplungsmittel aufweist. In bevorzugten Weiterbildungen weist die männliche Schnittstelleneinrichtung die zweiten mechanischen Kopplungsmittel auf und die weibliche Schnittstelleneinrichtung weist die ersten mechanischen Kopplungsmittel auf. Für diesen Fall weist vorzugsweise die weibliche Schnittstelleneinrichtung wenigstens eine Hinterschneidung auf, und die männliche Schnittstelleneinrichtung weist vorzugsweise wenigstens einen Bolzen auf, der in die Hinterschneidung eingreifen kann. Die Gestaltung, ob an der männlichen oder der weiblichen Schnittstelleneinrichtung die ersten oder zweiten mechanischen Kopplungsmittel ausgebildet sind, hängt maßgeblich vom Einsatzzweck ab und kann frei gewählt werden. Eine Einschränkung der Erfindung soll hierin nicht liegen.

Gemäß einem dritten Aspekt der Erfindung, wird die eingangs genannte Aufgabe bei einem Haltearm der eingangs genannten Art dadurch gelöst, dass die zweite Schnittstelle des Haltearms eine medizinische mechatronische männliche Schnittstelleneinrichtung nach einer der vorstehend beschriebenen bevorzugten Ausführungsformen der männlichen Schnittstelleneinrichtung gemäß dem ersten Aspekt der Erfindung aufweist. Alternativ weist der Haltearm die weibliche Schnittstelleneinrichtung nach einer der vorstehend beschriebenen bevorzugten Ausführungsformen der weiblichen Schnittstelleneinrichtung gemäß dem zweiten Aspekt der Erfindung auf. Es hat sich herausgestellt, dass es besonders vorteilhaft ist, die männliche Schnittstelleneinrichtung am Haltearm vorzusehen. Hierdurch können konstruktive Vorteile erzielt werden und die Stabilität und Steifigkeit kann erhöht sein.

Gemäß einem vierten Aspekt betrifft die Erfindung ein chirurgisches Assistenzsystem mit einer medizinischen weiblichen Schnittstelleneinrichtung nach einem der vorstehend beschriebenen bevorzugten Ausführungsformen der weiblichen Schnittstelleneinrichtung gemäß dem zweiten Aspekt der Erfindung. Auch hier ist alternativ vorgesehen, dass das chirurgische Assistenzsystem eine männliche Schnittstelleneinrichtung aufweist.

Ferner betrifft die Erfindung in einem fünften Aspekt ein System mit einem Haltearm der vorstehend beschriebenen Art und einem chirurgischen Assistenzsystem der vorstehend beschriebenen Art, das an einem Haltearm gekoppelt ist. Vorzugsweise ist das chirurgische Assistenzsystem als robotischer Manipulator ausgebildet. Vorzugsweise weist das System zwei oder mehr chirurgische Assistenzsysteme auf, von denen wenigstens eines robotisch ausgebildet ist und vorzugsweise wenigstens eines passiv ausgebildet ist. Hierdurch ist ein modulares System erreicht und ein Operateur kann je nach Aufgabenfeld ein anderes chirurgisches Assistenzsystem an den Haltearm koppeln.

Nachstehend wird die Erfindung anhand von Ausführungsbeispielen näher erläutert werden. Dabei zeigen:
- Figur 1: eine perspektivische Ansicht eines Haltearms mit einer männlichen Schnittstelleneinrichtung und einem chirurgischen Assistenzsystem mit einer weiblichen Schnittstelleneinrichtung, die aneinander gekoppelt sind;
- Figur 2: eine Detaildarstellung eines Haltearms mit einer männlichen Schnittstelleneinrichtung;
- Figur 3: eine perspektivische Ansicht einer medizinisch mechatronischen männlichen Schnittstelleneinrichtung gemäß der Erfindung;
- Figur 4: eine Frontalansicht der Schnittstelleneinrichtung gemäß Figur 3;
- Figur 5: eine Seitenansicht der Schnittstelleneinrichtung aus Figur 4;
- Figur 6: eine Draufsicht der Schnittstelleneinrichtung aus Figur 5;
- Figur 7: ein Gehäuse eines chirurgischen Assistenzsystems mit einer weiblichen Schnittstelleneinrichtung in perspektivischer Darstellung;
- Figur 8: eine Seitenansicht eines chirurgischen Assistenzsystems mit der weiblichen Schnittstelleneinrichtung, wobei das Gehäuse weggelassen ist;
- Figur 9: eine perspektivische Ansicht der medizinischen mechatronischen weiblichen Schnittstelleneinrichtung;
- Figur 10: eine Frontalansicht der Schnittstelleneinrichtung aus Figur 9;
- Figur 11: eine Seitenansicht der Schnittstelleneinrichtung aus Figur 10 mit betätigter Betätigungseinrichtung;
- Figur 12: eine Frontalansicht der Schnittstelleneinrichtung aus Figur 11;
- Figur 13: ein zweites Ausführungsbeispiel eines chirurgischen Assistenzsystems in perspektivischer Darstellung;
- Figur 14: ein drittes Ausführungsbeispiel eines chirurgischen Assistenzsystems in perspektivischer Darstellung;
- Figur 15: eine Rückansicht des chirurgischen Assistenzsystems aus Figur 13;
- Figuren 16a-16d: vier Ansichten eines zweiten Ausführungsbeispiels der männlichen Schnittstelleneinrichtung;
- Figur 17: eine Seitenansicht eines chirurgischen Assistenzsystems mit einer weiblichen Schnittstelleneinrichtung gemäß einem zweiten Ausführungsbeispiel; und
- Figur 18: das chirurgische Assistenzsystem aus Figur 17 im Schnitt A-A.

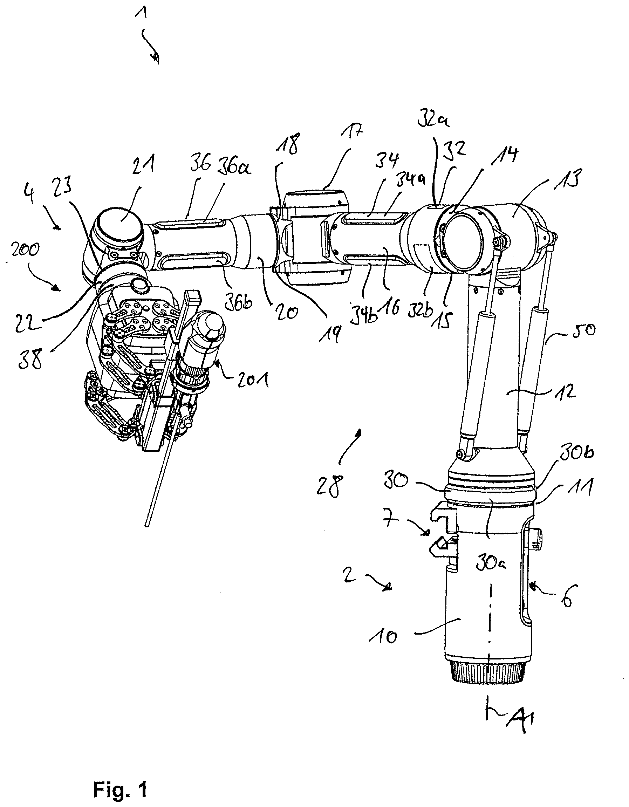

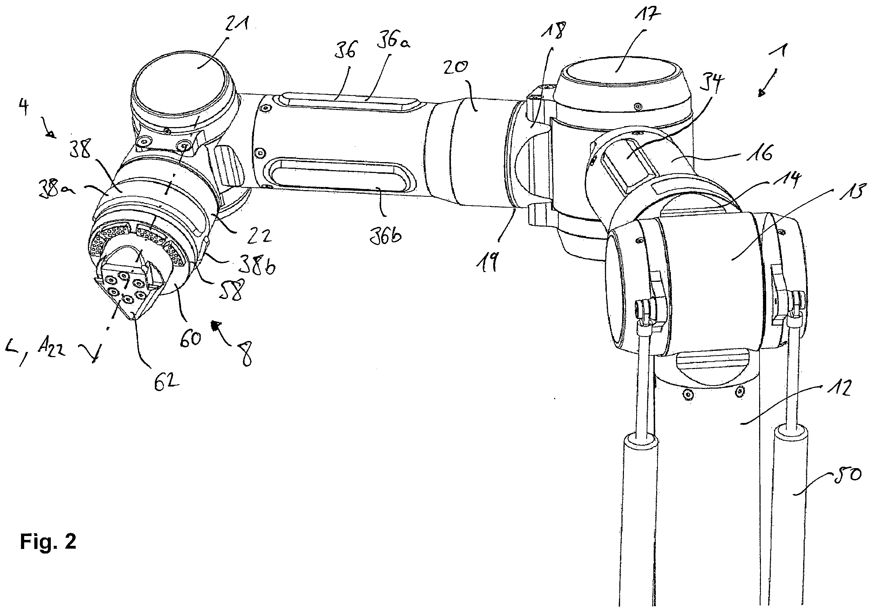

Figur 1 zeigt einen Haltearm 1 für medizinische Zwecke, insbesondere zum Halten eines chirurgischen mechatronischen Assistenzsystems 200 und/oder chirurgischen Instruments. Der Haltearm 1 weist ein proximales Ende 2 und ein distales Ende 4 auf. An dem proximalen Ende 2 sind eine erste Schnittstelle 6 und eine mechanische Schnittstelle 7 ausgebildet. Die Schnittstelle 7 dient dazu, den Haltearm 1 an einer Basis, wie insbesondere an einem Operationstisch, zu befestigen. Die Schnittstelle 6 dient zur Übergabe von Energie sowie zur Kopplung des Haltearms 1 mit einer externen Steuereinheit. An dem distalen Ende 4 ist eine zweite Schnittstelle 8 (vgl. Fig. 2) vorgesehen, über die ein passives und ein mechatronisches (aktives) Assistenzsystem, wie insbesondere eine Manipulatorvorrichtung, an den Haltearm 1 koppelbar ist. Vorzugsweise wird hier eine Manipulatorvorrichtung 200 zum Halten und Manipulieren eines Endoskops 201 angeordnet.

Der Haltearm 1 gemäß Figur 1 weist sieben Armsegmente 10, 12, 14, 16, 18, 20, 22 auf, die jeweils im Wesentlichen stabförmig sind und bis auf das letzte Armsegment 22 alle im Wesentlichen eine gleiche Länge aufweisen. Die sieben Armsegmente 10, 12, 14, 16, 18, 20, 22 sind jeweils mit Gelenken 11, 13, 15, 17, 19, 21, 23 miteinander gekoppelt, wobei das nullte Gelenk 11 den Haltearm 1 mit der Basis koppelt. Die Gelenke 13, 15, 17, 19, 21, 23 sind gemäß diesem Ausführungsbeispiel sämtlich als rotatorische Gelenke mit jeweils einem Freiheitsgrad ausgebildet. Gemäß diesem Ausführungsbeispiel ist dem nullten Segment 10 das nullte Gelenk 11 zugeordnet, dem ersten Armsegment 12 das erste Gelenk 13 zugeordnet, dem zweiten Armsegment 14 das zweite Gelenk 15 zugeordnet, dem dritten Armsegment 16 das dritte Gelenk 17 zugeordnet, dem vierten Armgelenk 18 das vierte Gelenk 19 zugeordnet, dem fünften Armsegment 20 das fünfte Gelenk 21 zugeordnet, und dem sechsten Armsegment 22 ist das sechste Gelenk 23 zugeordnet. Die Gelenke 11, 13, 15, 17, 19, 21, 23 weisen jeweils Schwenkachsen auf, wobei jeweils benachbarte Gelenke Schwenkachsen haben, die senkrecht zueinander sind. Hierdurch wird eine einfache Positionierung des distalen Endes 4 im Raum erreicht.

Der Haltearm 1 gemäß Figur 1 weist ferner eine Bedieneinrichtung 28 auf. Mittels der Bedieneinrichtung 28 ist der Haltearm 1 in eine gewünschte Pose verbringbar, wobei die Bedieneinrichtung 28 dazu eingerichtet ist, bei Kontakt zwischen einer Bedienperson und einem der sieben Armsegmente das zugeordnete Gelenk und/oder weitere Gelenke je nach Konfiguration und Parametrisierung der Bedieneinrichtung 28 freizugeben. Dazu weist die Bedieneinrichtung 28 gemäß diesem Ausführungsbeispiel fünf Kontaktabschnitte 30, 32, 34, 36, 38 auf.

Ferner ist gemäß diesem Ausführungsbeispiel vorgesehen, dass jedes Kontaktmittel 30, 32, 34, 36, 38, 40, 42 jeweils zwei im Wesentlichen gegenüberliegend angeordnete Kontaktmittelelemente 30a, 30b, 32a, 32b, 34a, 34b, 36a, 36b aufweist. Die Kontaktmittel 30, 32, 34, 36, 38, 40, 42 dienen dazu, einen Kontakt eines Bedieners mit dem entsprechenden Armsegment 10, 12, 14, 16, 18, 20, 22 zu erfassen. Beim Ergreifen eines Armsegments 10, 12, 14, 16, 18, 20, 22 kommt der Bediener in Kontakt mit beiden Kontaktmittelelementen 30a, 30b bis 36a, 36b, und nur bei Kontakt mit beiden Kontaktmittelelementen 30a, 30b bis 36a, 36b eines Kontaktmittels 30 bis 38 wird das zugeordnete Gelenk freigegeben. Das heißt, beim Ergreifen des ersten Armsegments 10 und gleichzeitigem Inkontaktkommen mit den beiden Kontaktmittelelementen 32a, 32b wird durch die Bedieneinrichtung 28 das erste Gelenk 11 freigegeben. Dadurch ist es möglich, dass der Bediener den Haltearm 1 beziehungsweise die Armsegmente 12 bis 22 um die Achse A₁ verschwenken kann. Bei Loslassen eines der beiden oder beider Kontaktmittelelemente 32a, 32b wird das Gelenk 11 wieder arretiert, und ein Verschwenken um die Achse A₁ ist nicht mehr möglich. Auch bei einer unbeabsichtigten Berührung nur eines der beiden Kontaktmittelelemente 32a, 32b, beispielsweise mit einem Arm oder Ellenbogen des Bedieners, wird das Gelenk 11 nicht freigegeben, und der Haltearm 1 bleibt im arretierten Zustand und hält seine Pose.

In den dargestellten Ausführungsbeispielen ist teilweise auch nur ein Kontaktmittel für zwei oder mehr Gelenke vorgesehen. So wird beispielsweise bei Betätigung des Kontaktmittels 38 sowohl das Gelenk 23 als auch das Gelenk 21 freigegeben.

Die Bedieneinrichtung 28 kann dazu einen Controller oder Mikroprozessor aufweisen, der dazu eingerichtet ist, einen Kontakt zwischen Kontaktmittelelementen 30a, 30b bis 42a, 42b zu erfassen und in elektrische Signale zu übertragen.

Die Kontaktmittelemente 30a, 30b bis 36a, 36b sind gemäß diesem Ausführungsbeispiel als berührungsempfindliche Sensoren ausgebildet und erfassen einen Druck eines Kontakts zwischen dem Bediener und dem entsprechenden Kontaktmittelelement 30a, 30b bis 36a, 36b. Vorzugsweise sind die Kontaktmittelelemente 30a, 30b bis 36a, 36b als kapazitive berührungsempfindliche Sensoren ausgebildet.

Bei dem dargestellten Haltearm 1 ist es auch möglich, dass ein Bediener zwei Armsegmente, beispielsweise das Armsegment 14 und das Armsegment 18, gleichzeitig ergreift und so gleichzeitig die Kontaktmittelelemente 34a, 34b und 38a, 38b kontaktiert. Folglich werden die Gelenke 15 und 19 freigegeben, und ein Verschwenken ist möglich. Bei dieser gleichzeitigen Freigabe ist es möglich, eine Winkelorientierung der Armsegmente 18 und 20 im Raum beizubehalten, während nur die Armsegmente 34, 36 verschwenkt werden. So ist auch eine translatorische Bewegung des distalen Endes 4 möglich. In einer bevorzugten Ausgestaltung des Haltearms werden bei dem gleichzeitigen Kontaktieren von zwei Armsegmenten, gemäß diesem Beispiel der Armsegmente 14 und 18 nicht die Gelenke 15 und 19 freigegeben, sondern alle zwischen diesen Armsegmenten 14 und 18 liegenden Gelenke, also gemäß diesem Ausführungsbeispiel die Gelenke 17 und 19. Das Gelenk 15 bleibt arretiert. Dies ist eine besonders intuitive Bedienung des Haltearms. Entsprechend werden, beispielsweise bei dem Kontakt zwischen Bediener und den Haltearmsegmenten 12 und 20, die Gelenke 15, 17, 19 und 21 freigegeben.

Weiterhin ist in der Figur 1 zu erkennen, dass der Haltearm 1 über eine Gewichtskompensationseinrichtung 50 verfügt. Die Gewichtskompensationseinrichtung 50 weist gemäß diesem Ausführungsbeispiel ein Gasdruckfederelement auf, welches mit dem Armsegment 14 und dem Armsegment 12 gekoppelt ist. Alternativ kann die Gewichtskompensationseinrichtung auch einen Seilzug und/oder ein ausbalanciertes Gegengewicht aufweisen. Bei dem Haltearm 1 gemäß Figur 1 lastet auf dem Gelenk 15 um seine Rotationsachse A2 das größte Moment. Folglich ist es bevorzugt, genau dieses Gelenk 15 mittels der Gewichtskompensationseinrichtung 50 abzustützen. Bei Freigabe des Gelenks 15, durch Kontaktieren des Armsegments 14, wird also ein Gewicht, welches auf dem Armsegment 14 lastet, aufgrund der weiteren Armsegmente 16, 18, 20, 22 und eines an der Schnittstelle 8 angeordneten Manipulators durch die Gewichtskompensationseinrichtung 50 abgestützt, sodass das distale Ende 4 bei Ergreifen des Segments 14 nicht sofort "absackt".

Der grundsätzliche Aufbau eines solchen Haltearms ist beispielsweise in DE 10 2014 016 824 A1, DE 10 2014 019 752 A1 sowie auch in DE 10 2015 104 810 A1 beschrieben. Auf diese Dokumente wird vollumfänglich Bezug genommen und der Inhalt durch Referenz hierin mit einbezogen.

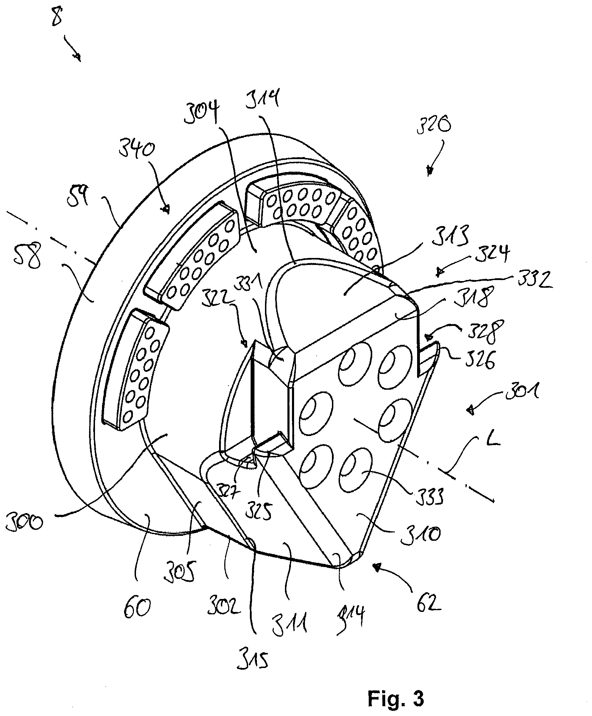

An dem distalen Ende 4 ist die medizinische mechatronische männliche Schnittstelleneinrichtung 8 gemäß der Erfindung vorgesehen (Figur 2). Diese wird mit Bezug auf die Figuren 3 bis 6 noch im Detail erläutert werden. Wie in Figur 2 zu sehen, ist die Zentralachse L der männlichen Schnittstelleneinrichtung 8 koaxial mit der Achse A22 des letzten Armsegments 22, das das distale Ende 4 des Haltearms 1 bildet.

Die medizinische mechatronische männliche Schnittstelleneinrichtung 8 weist eine Basis 58 auf, über die die Schnittstelleneinrichtung 8 an dem letzten Armsegment 22 befestigt ist. Die Basis 58 hat eine Stirnseite 60, von der sich ein Funktionskörper 62 erstreckt, dessen Zentralachse die Achse L ist. In anderen Ausführungsbeispielen kann die Schnittstelleneinrichtung 8 auch an jeglicher anderer Haltestruktur befestigt sein, wie beispielsweise einem Stativ, einem Roboterarm, einem chirurgischen Instrument, einem OP-Tisch, einem Deckenstativ, oder an jeglichem anderen Halter oder dergleichen.

Die Basis 58 hat in dieser Ausführungsform eine etwa scheibenförmige Struktur und ist in sich im Wesentlichen zylindrisch gebildet. Die Basis 58 kann aber auch jegliche andere Form haben und dient insbesondere dazu, einen Anschluss an die Haltestruktur zu bilden. Insofern ist es sinnvoll, wenn die Basis 58 eine Umfangskontur 59 hat, die in etwa der des entsprechenden Montageabschnitts der Haltestruktur entspricht, wie hier insbesondere der Umfangsstruktur des letzten Armsegments 22. So ist ein glatter Übergang zwischen der Basis 58 und dem letzten Armsegment 22 geschaffen. Sollte beispielsweise das letzte Armsegment 22 eine ovale Struktur haben, wäre es bevorzugt, dass auch die Basis 58 einen ovalen Umfang 59 hat. Auf diese Weise lässt sich auch die Struktur des Funktionskörpers 62 unabhängig von der Struktur der Basis 58 schaffen.

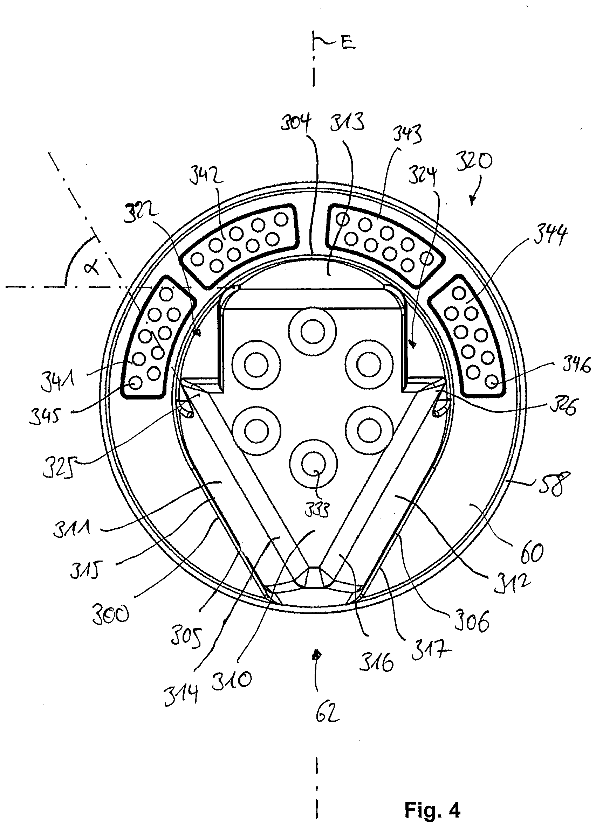

Der Funktionskörper 62 erstreckt sich in dieser Ausführungsform senkrecht und axial von der Basis 58 bzw. der Stirnseite 60. Der Funktionskörper 62 hat einen Grundkörper 300. Der Grundkörper 300 ist im Grundsatz zylindrisch entlang der Längsachse L, weist aber eine sich radial erstreckende Nase 302 auf. Der zylindrische Abschnitt 304 geht glatt in die Nase 302 über, die mit ihren Seiten 305, 306 (vgl. auch Fig. 4) tangential zum zylindrischen Teil 304 des Grundkörpers 300 ausläuft. Der zylindrische Abschnitt 304 erstreckt sich umfänglich etwa um 180°, vorzugsweise etwa um 200° um die Längsachse L.

Der Funktionskörper 62 weist ferner eine Stirnseite 310 auf, die in diesem Ausführungsbeispiel im Wesentlichen senkrecht zur Längsachse L ausgerichtet ist. Schräg zur Längsachse L und schräg zueinander verlaufen eine erste Funktionsfläche 311, eine zweite Funktionsfläche 312 und eine dritte Funktionsfläche 313. Die ersten, zweiten und dritten Funktionsflächen 311, 312, 313 sind in sich eben, aber jeweils gewinkelt zueinander und gewinkelt zur Zentralachse L. Die Funktionsflächen 311, 312, 313 schließen bezogen auf eine Ebene senkrecht zur Zentralachse L jeweils einen Winkel *α* ein (vgl. Fig. 4; dort ist nur beispielhaft der Winkel *α* zwischen der ersten Funktionsfläche 311 und der dritten Funktionsfläche 313 angetragen), der in einem Bereich von etwa 60° liegt. Der Winkel *α* zwischen den weiteren Funktionsflächen, nämlich der Winkel zwischen der zweiten Funktionsfläche 312 und der dritten Funktionsfläche 313 sowie der ersten Funktionsfläche 311 und der zweiten Funktionsfläche 312 ist identisch zum Winkel *α*.

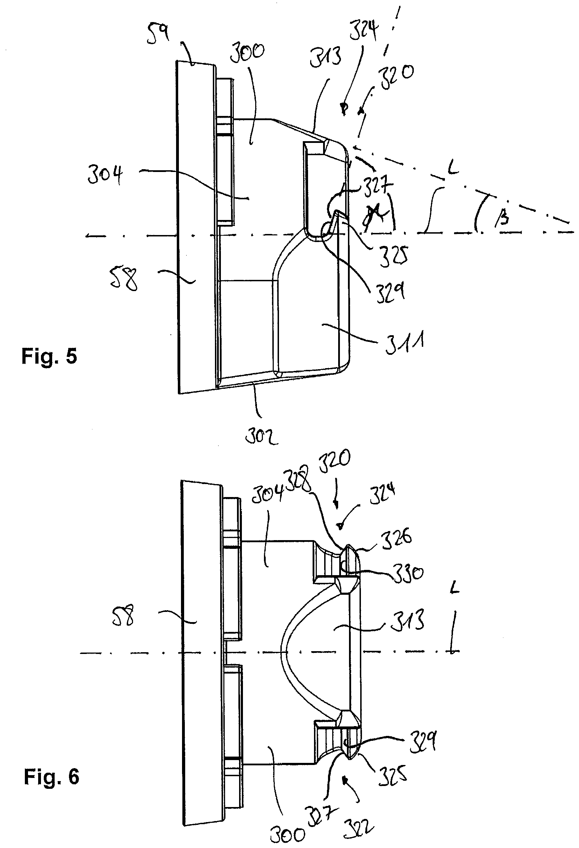

Weiterhin sind die ersten, zweiten und dritten Funktionsflächen 311, 312, 313 zur Zentralachse L geneigt (vgl. Fig. 5). In Figur 5 ist nur die Neigung der dritten Funktionsfläche 313 zur Zentralachse L angezeichnet, die einen Winkel *β* mit dieser einschließt. Die Neigung der ersten Funktionsflächen 311 und der zweiten Funktionsfläche 312 zur Zentralachse L ist vorzugsweise identische ausgebildet. Die dritte Funktionsfläche 313 schließt mit der Zentralachse L einen Winkel *β* in einem Bereich von wenigstens 20° ein. In diesem Ausführungsbeispiel beträgt der Winkel *β* in etwa 30°. Die Neigung der ersten Funktionsflächen 311 und der zweiten Funktionsfläche 312 ist in diesem Ausführungsbeispiel identisch ausgebildet und beträgt ebenfalls in etwa 30° bezogen auf die Zentralachse L.

Auch die ersten, zweiten und dritten Funktionsflächen 311, 312, 313 gehen im Wesentlichen eben und knickfrei einerseits in die Stirnseite 310 über und andererseits in die Umfangsfläche des Grundkörpers 300. Insofern läuft die erste Funktionsfläche 311 einerseits bei einem Abschnitt 314 in die Stirnseite 310 aus und an einem Abschnitt 315 in den Grundkörper 300. Ebenso läuft die zweite Funktionsfläche 312 bei einem Abschnitt 316 in die Stirnseite 310 aus und bei einem Abschnitt 317 in den Grundkörper 300. Die dritte Funktionsfläche 313 läuft in einem Abschnitt 318 in die Stirnseite 310 aus, bei einem Abschnitt 319 in die Umfangsfläche des Grundkörpers 300. Jeder der Abschnitte 314, 315, 316, 317, 318, 319 weist einen Radius auf, der wenigstens 0,5 mm beträgt, vorzugsweise wenigstens 1 mm. Hierdurch sind glatte Übergänge geschaffen, wodurch einerseits das Verletzungsrisiko verringert werden kann, als auch die Reinigung der männlichen Schnittstelleneinrichtung 8 vereinfacht ist.

Die männliche Schnittstelleneinrichtung 8 weist weiterhin mechanische Kopplungsmittel 320 auf, die gemäß dieser Ausführungsform eine erste Ausnehmung 322 und eine zweite Ausnehmung 324 aufweisen. Die Ausnehmungen 322, 324 sind in etwa senkrecht zur Zentralachse L eingebracht und schneiden teilweise in die ersten und zweiten Funktionsflächen 311, 312 ein. Sie erstrecken sich auch durch einen Teil der Stirnseite 310, wodurch diese eine etwa pfeilförmige Kontur erhält (siehe insbesondere Fig. 3 und 4).

Jeder der Ausnehmungen 322, 324 ist zur Stirnseite 310 hin durch einen Absatz 325, 326 begrenzt, der eine Hinterschneidung 327, 328 bildet. Die nach innen gerichtete Flanke 329, 330 der Hinterschneidung 327, 328 ist bezogen auf die Zentralachse L schräg ausgebildet und schließt einen Winkel *γ* ein (siehe Fig. 5). Der Winkel *γ* ist kleiner als 90°, vorzugsweise 80°, aber vorzugsweise größer als 45°. Der Winkel *γ* bildet eine Einführschräge für die Hinterschneidung 327, 328, wie dies später noch erläutert werden wird.

Weiterhin sind an den Ausnehmungen 322, 324, Einführschrägen 331, 332 vorgesehen, die in die dritte Funktionsfläche 313 auslaufen. Auch diese werden später noch näher erläutert.

In der Stirnseite 310 sind ferner sechs Bohrungen 333 (in Fig. 3 und 4 ist jeweils nur eine mit Bezugszeichen versehen) vorgesehen. Über diese Bohrungen kann die männliche Schnittstelleneinrichtung 8 mit einer entsprechenden Haltestruktur verbunden werden.

Die männliche Schnittstelleneinrichtung 8 weist darüber hinaus eine erste elektronische Schnittstelle 340 auf. Die erste elektronische Schnittstelle 340 weist gemäß diesem Ausführungsbeispiel vier Blöcke 341, 342, 343, 344 auf, die jeweils eine Mehrzahl an elektrischen Kontakten, in diesem Ausführungsbeispiel jeweils neun elektrische Kontakte aufweisen. Die ersten, zweiten, dritten und vierten Blöcke 341, 342, 343, 344 sind hier als weibliche Stecker der elektronischen Schnittstelle 340 ausgebildet. Sie sind an ihrem Rand gegenüber der Stirnseite 60 abgedichtet, aber vorzugsweise federnd gelagert. Die Steckrichtung und somit die Kupplungsrichtung der elektronischen Schnittstelle 340 ist parallel zur Zentralachse L.

Insgesamt ist die männliche Schnittstelleneinrichtung in ihrer äußeren Struktur spiegelsymmetrisch ausgebildet, zur Ebene E (siehe Fig. 4). Der Funktionskörper 62 bildet einen dreiseitigen Pyramidenstumpf. Hierdurch wird eine besonders einfache Zentrierung erreicht. Die Nase 302 des Grundkörpers 300 bietet einen zusätzlichen Verdrehschutz und Verdrehsicherung bezogen auf die Zentralachse L.

Die erste elektronische Schnittstelle 340 weist einen ersten und einen zweiten Erdungskontakt 345, 346 auf. Der erste Erdungskontakt 345 ist in dem ersten Block 341 angeordnet und der zweite Erdungskontakt 346 in einem zweiten Block 344. Die Erdungskontakte 345, 346 sind diejenigen Kontakte, die am weitesten voneinander entfernt sind. Dies bedeutet, auch bei einem leichten Verkippen der weiblichen Schnittstelleneinrichtung gegenüber der männlichen Schnittstelleneinrichtung 8 beim Kuppeln führt stets dazu, dass einer der Erdungskontakte 345, 346 zuerst in Kontakt kommt, während erst anschließend die übrigen elektrischen Kontakte der Blöcke 341, 342, 343, 344 geschlossen werden.

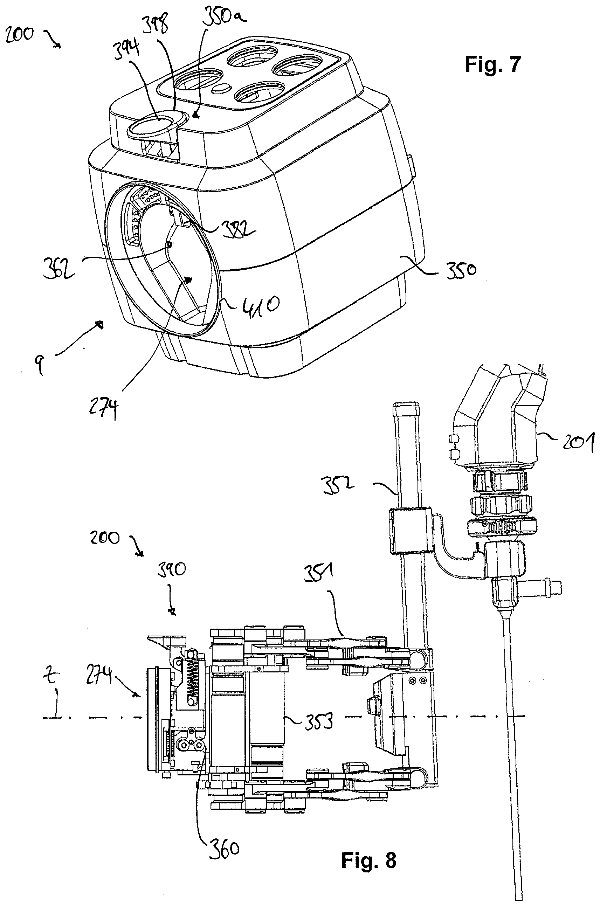

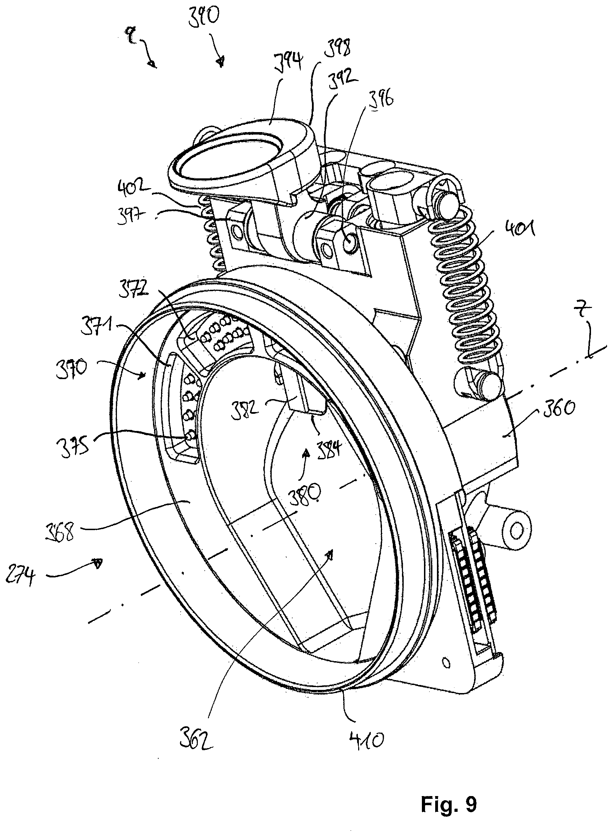

Im Folgenden zeigen zunächst die Figuren 7 und 8 einen Überblick über die medizinische weibliche Schnittstelleneinrichtung 9, während diese dann mit Bezug auf die Figuren 9 bis 11 im Detail beschrieben werden wird.

In Figur 7 ist zunächst ein Gehäuse 350 eines chirurgischen Assistenzsystems 200, nämlich einem mechatronischen Manipulator 200 wie in Figur 1 gezeigt perspektivisch dargestellt. Bei der Darstellung in Figur 7 ist die gesamte Kinematik weggelassen, aber die medizinische weibliche Schnittstelleneinrichtung 9 an einer Seite des Gehäuses 350 ist zu sehen.

Im Gegensatz dazu ist in Figur 8 das Gehäuse 350 des chirurgischen Assistenzsystems 200, nämlich dem mechatronischen Manipulator 200 weggelassen, aber die Kinematik 351 samt Linearantrieb 352 und Endoskop 201 ist zu sehen. Der mechatronische Manipulator 200 weist im Inneren einen Antrieb 353 auf, der über die weibliche Schnittstelleneinrichtung 9 mit Stellsignalen und elektrischer Energie versorgt werden kann.

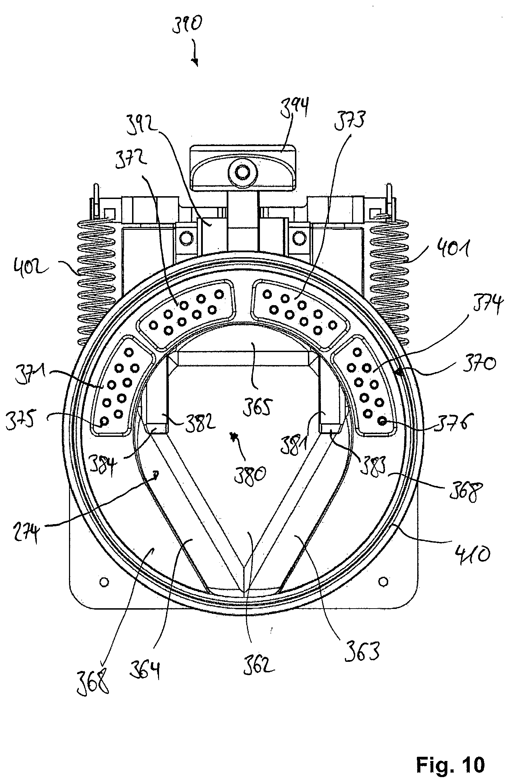

Die weibliche Schnittstelleneinrichtung 9 zum Koppeln eines chirurgischen Assistenzsystems an einer Haltstruktur, wie insbesondere den Haltearm 1, ist zum Koppeln mit er männlichen Schnittstelleneinrichtung 8 ausgelegt, wie sie vorstehend mit Bezug auf die Figuren 1 bis 6 erläutert wurde. Die weibliche Schnittstelleneinrichtung 9 weist einen Rahmen 360 auf, der eine Struktur für die Schnittstelleneinrichtung 9 bildet. Die Schnittstelleneinrichtung 9 weist ferner eine zentrale Ausnehmung 274 zur Aufnahme des Funktionskörpers 62 auf, der in dem Rahmen 360 ausgebildet ist. Die zentrale Ausnehmung 274 hat eine Zentralachse Z, die im gekoppelten Zustand der weiblichen Schnittstelleneinrichtung 9 an die männliche Schnittstelleneinrichtung 8 koaxial mit der Achse L de r männlichen Schnittstelleneinrichtung 8 verläuft. Die zentrale Ausnehmung 274 hat eine Zentriereinrichtung 362, wobei die Zentriereinrichtung 362 eine weibliche erste Funktionsfläche 363, eine zweite weibliche Funktionsfläche 364, und eine dritte weibliche Funktionsfläche 365 aufweist. Diese drei Funktionsflächen 363, 364, 365 sind am besten in Figur 10 zu sehen. Die Funktionsflächen 363, 364, 365 sind korrespondierend zu den ersten, zweiten und dritten Funktionsflächen 311, 312, 313 der männlichen Schnittstelleneinrichtung 8 ausgebildet. Im angekuppelten Zustand liegen die Funktionsflächen 363, 364, 365 entsprechend an den Funktionsflächen 311, 312, 313 an. Sie weisen daher auch dieselben Neigungen, oder ähnlichen Neigungen auf, wie die ersten, zweiten und dritten Funktionsflächen 311, 312, 313.

Nach außen gerichtet weist die weibliche Schnittstelleneinrichtung 9 eine Stirnseite 368 auf, die mit der Stirnseite 60 der männlichen Schnittstelleneinrichtung 8 korrespondiert. An der Stirnseite 386 ist eine zweite elektronische Schnittstelle 390 ausgebildet, die zu der ersten elektronischen Schnittstelle 340 korrespondiert. In diesem Ausführungsbeispiel weist die zweite elektronische Schnittstelle 370 daher erste, zweite, dritte und vierte Vertiefungen 371, 372, 373, 374 auf, in denen eine Mehrzahl elektrischer Kontakte (nicht mit Bezugszeichen versehen) vorgesehen sind, nämlich jeweils neun elektrische Kontakte, die mit den elektrischen Kontakten der ersten elektronischen Schnittstelle 340 korrespondieren. Insofern weist auch die zweite elektronische Schnittstelle 370 erste und zweite Erdungskontakte 375, 376 auf, die mit den Erdungskontakten 345, 346 der ersten elektronischen Schnittstelle 340 korrespondieren. Dies ist im vorliegenden Fall dadurch gelöst, dass die axiale Länge, in Richtung der Zentralachse Z der Erdungskontakte 375, 376 etwas länger ist, als die axiale Länge der übrigen elektrischen Kontakte. Hierdurch kann sichergestellt werden, dass der Kontaktschluss zwischen den entsprechenden Erdungskontakten 345, 346, 375, 376 zuerst erfolgt, und erst anschließend der Kontakt der übrigen Kontakte geschlossen wird.

Die weibliche Schnittstelleneinrichtung 9 weist darüber hinaus zweite mechanische Kopplungsmittel 380 auf. Die zweiten mechanischen Kopplungsmittel 380 sind zum form- und/oder kraftschlüssigen Koppeln mit den ersten mechanischen Kopplungsmitteln 320 der männlichen Schnittstelleneinrichtung 8 vorgesehen. Hierzu korrespondieren die zweiten mechanischen Kopplungsmittel 380 mit den ersten mechanischen Kopplungsmitteln 320. Die zweiten mechanischen Kopplungsmittel 380 umfassen in dieser Ausführungsform einen ersten Bolzen 381 und einen zweiten Bolzen 382, die sich im Wesentlichen senkrecht zur Zentralachse Z in die zentrale Ausnehmung 274 erstrecken. In den Figuren 7, 9 und 10 sind die ersten und zweiten Bolzen 381, 382 in der arretierten Stellung gezeigt. Die ersten und zweiten Bolzen 381, 382 weisen an ihrer Stirnseite, das heißt am freien axialen Enden, eine Abschrägung 383, 384 auf. Diese Abschrägung 383, 384 korrespondiert wenigstens abschnittsweise mit der Flanke 329, 330 der Hinterschneidung 327, 329 der ersten mechanischen Kopplungsmittel 320.

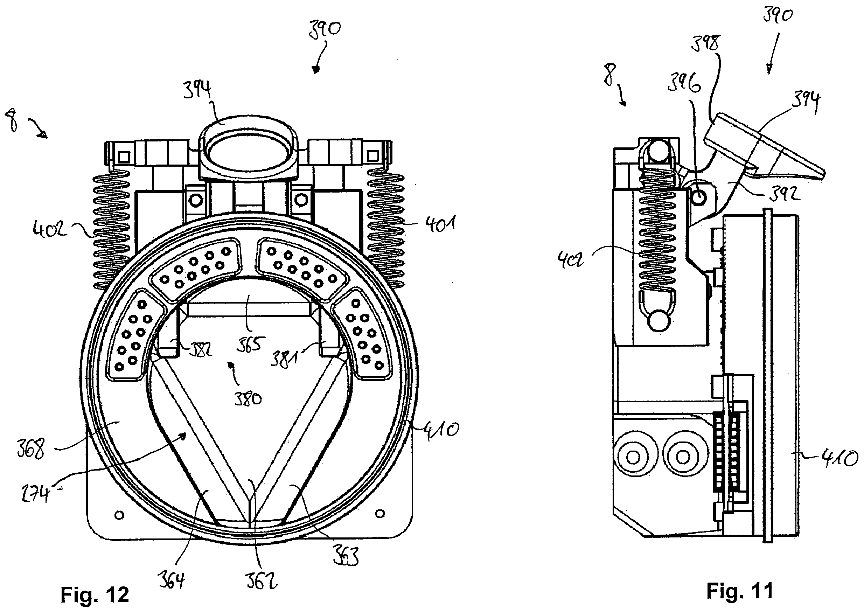

Die ersten und zweiten Bolzen 381, 382 sind gemeinsam über eine Betätigungseinrichtung 390 von der Arretierstellung (Figuren 7, 9, 10) in eine Freigabestellung (Figuren 11 und 12) verbringbar. Dazu sind die ersten und zweiten Bolzen 381, 382 an einem gemeinsamen Joch oder Gabel 392 gelenkig angelenkt, wobei sich von dem Joch 392 ein Betätigungsknopf 394 erstreckt. Der Betätigungsknopf 394 ist einstückig mit dem Joch verbunden. Das Joch 392 und somit auch der Betätigungsknopf 394 sind schwenkbar um eine Achse 396 in einem Stützabschnitt 397 des Rahmens 360 gelagert. Durch Verschwenken des Drucktasters 394 (vgl. Figuren 11 und 12) werden die ersten und zweiten Bolzen 381, 382 angehoben, das heißt von der Zentralachse Z entfernt und wenigstens teilweise aus der zentralen Ausnehmung 274 herausgezogen, sodass in diesem freigegebenen Zustand (Figuren 11, 12) die männliche Schnittstelleneinrichtung 8 mit dem Funktionskörper 92 in die zentrale Ausnehmung 274 der weiblichen Schnittstelleneinrichtung 9 eingeführt werden kann. Das heißt, die ersten und zweiten Bolzen 381, 382 sind jedenfalls soweit angehoben, dass sie axial über die Vorsprünge 325, 326 gelangen können. Wird dann der Drucktaster 394 wieder losgelassen, wird dieser durch eine Federvorspannung, die von ersten und zweiten Zugfedern 401, 402 bereitgestellt wird, in die Arretierstellung verbracht. Das heißt, die zweiten mechanischen Kopplungsmittel 380 sind in die Arretierstellung vorgespannt. Beim Arretieren werden die ersten und zweiten Bolzen 381, 382mit Bezug auf Fig. 5 von oben in die Ausnehmungen 322, 324 eingeführt und gelangen hinter die Hinterschneidungen 327, 328. Hier können nun die Flanken 329, 330 und die Anschrägungen 383,384 zum Einsatz kommen. Sind die männlichen und weiblichen Schnittstellen 8, 9 nicht weit genug gefügt, kommen die ersten und zweiten Bolzen 381, 382 zunächst mit ihren Anschrägungen 383, 384 in Kontakt mit einem mit Bezug auf Figur 5 oberen Abschnitt der Vorsprünge 325, 326. Die Bolzen 381, 382 rutschen dann an der schrägen Flanke 329, 330 entlang in die Hinterschneidung hinein. Hierdurch kann sichergestellt werden, dass die weibliche Schnittstelleneinrichtung 9 an die männliche Schnittstelleneinrichtung 8 herangezogen werden kann.

Es kann vorgesehen sein, dass die Geometrie der Funktionsflächen 311, 312, 313, 363, 364, 365 und die Positionierung der Hinterschneidungen 327, 328 sowie der ersten und zweiten Bolzen 381, 382 so gewählt ist, dass die ersten, zweiten Bolzen 381, 382 stets an den Flanken 329, 330 anliegen, und somit stets eine Zugkraft auf die männlichen und weiblichen Schnittstelleneinrichtungen 8, 9 ausüben, um eine feste Kopplung zu erreichen. Diese Kopplung ist dann spielfrei. Aufgrund der statischen Bestimmtheit der Kontakte der jeweils Funktionsflächen 311, 312, 313 und 363, 364, 365 ist der Kontakt des Funktionskörpers 62 mit der zentralen Ausnehmung 274 auch statisch bestimmt.

Der Druckknopf 394 weist einen Rücken 398 auf, der in der arretierten Position so ausgerichtet ist, dass er von dem Abschnitt 350a des Gehäuses 350 verdeckt wird. Befindet sich der Druckknopf 394 in der verschwenkten Stellung und sind somit die zweiten mechanischen Kopplungsmittel 380 in der freigegebenen Position, ist der Rücken 398 sichtbar. Der Rücken 398 weist vorzugsweise eine Signalfarbe auf, beispielsweise eine rote Farbe. Hierdurch ist für den Bediener leicht zu erkennen, wenn sich der Druckknopf 394 in der freigegebenen Position befindet (Fig. 11) und somit eine sichere Kopplung der weiblichen Schnittstelleneinrichtung 9 und der männlichen Schnittstelleneinrichtung 8 nicht gewährleistet ist.

Alternativ zu dieser optischen Überprüfung mittels des Rückens 398 können andere Sicherungseinheiten oder Einrichtungen vorgesehen sein. So kann beispielsweise vorgesehen sein, dass durch das Verbringen der ersten und zweiten Bolzen 381, 382 in die Arretierposition ein elektrischer Kontakt geschlossen wird oder geöffnet wird und in Abhängigkeit davon eine Signallampe leuchtet. Ebenso ist es möglich, dass auf ähnliche Weise ein akustisches Signal ausgegeben wird. Weiterhin kann vorgesehen sein, dass an wenigstens einer der Flächen 311, 312, 313, 363, 364, 365 ein oder mehrere Drucksensoren vorgesehen sind, die ein entsprechendes Signal bereitstellen, wenn ein Schwellwert über- und/oder unterschritten wird. Wird beispielsweise festgestellt, dass ein Schwellwert unterschritten wird, ist eine Kopplung der männlichen Schnittstelleneinrichtung 8 und der weiblichen Schnittstelleneinrichtung 9 nicht ausreichend und ein entsprechendes Signal wird vorzugsweise ausgegeben. Um die zentrale Ausnehmung 274 der weiblichen Schnittstelleneinrichtung 9 herum ist ein Kragen 410 ausgebildet. Dieser Kragen 410 erstreckt sich im Wesentlichen axial von der Stirnseite 368 und umschließt im gekoppelten Zustand die Mantelflächen 359 der Basis 358 der männlichen Schnittstelleneinrichtung 8. Hierdurch wird eine weitere Abdichtung erreicht. Der Kragen 410 kann hierzu beispielsweise aus einem flexiblen Material, wie beispielsweise mit Kunststoff oder Gummi gebildet sein.

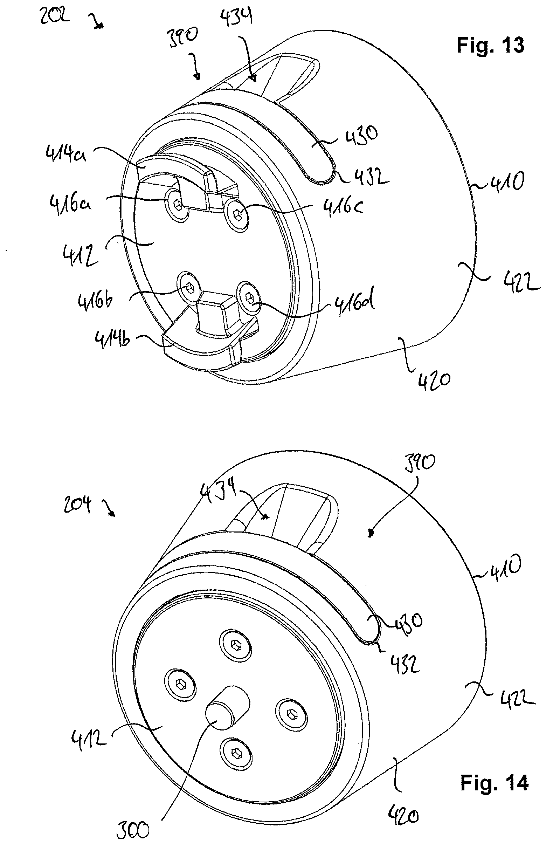

Die Figuren 13 bis 15 illustrieren nun weitere Ausführungsbeispiele von chirurgischen Assistenzsystemen. Gleiche und ähnliche Elemente der Figuren 13 bis 15 sind mit gleichen Bezugszeichen wie in den vorherigen Ausführungsbeispielen bezeichnet und insofern wird vollumfänglich Bezug genommen.

Die Figur 13 zeigt ein chirurgisches Assistenzsystem 202 und die Figur 14 ein chirurgisches Assistenzsystem 204. Beide chirurgischen Assistenzsysteme 202, 204 sind hier als Kappen ausgebildet und insofern passiv. Das chirurgische Assistenzsystem 202 unterscheidet sich von dem chirurgischen Assistenzsystem 204 durch seine Stirnfläche 412. Von der Stirnfläche 412 erstrecken sich bei dem chirurgischen Assistenzsystem 202 erste und zweite Formschlusselemente 414a, 414b, die zur Aufnahme eines medizinischen Instruments dienen können. Ein solches medizinisches Instrument kann insbesondere als Ankopplungsstück für ein Endoskop, eine Klemme, ein Laparoskop oder dergleichen dienen.

Bei dem Ausführungsbeispiel in der Figur 14 weist das chirurgische Assistenzsystem 204 anstelle der ersten und zweiten Formschlusselemente 414a, 414b einen Gewindeschaft 300 auf, der ebenfalls dazu dient, dass an diesem medizinische Instrumente aufgeschraubt werden können.

Wie sich auch aus den Figuren 13 und 14 ergibt, sind die Stirnseiten 412 im Wesentlichen plattenförmig ausgebildet und mittels vier Schrauben 416a, 416b, 416c, 416d mit einem Grundkörper 420 des chirurgischen Assistenzsystems 202, 204 verschraubt. Insofern können an demselben Grundkörper 420 verschiedene Stirnflächen 412 aufgeschraubt werden. Hierdurch ist eine Vielzahl an verschiedenen Varianten denkbar. Vorzugsweise sind an solchen Stirnflächen 412 beispielsweise Normschnittstellen für Werkzeuge, Instrumente und dergleichen vorgesehen, oder herstellerspezifische Schnittstellen, zur Aufnahme von herstellerspezifischen Instrumenten.

Der Grundkörper 420 ist an seiner Außenfläche 422 im Wesentlichen kegelstumpfförmig gebildet und weist in seinem Inneren die weibliche Schnittstelleneinrichtung 9 auf. Der Grundkörper 420 ist vorzugsweise insgesamt aus einem Kunststoff gebildet.

Im Unterschied zu dem chirurgischen Assistenzsystem 200 des vorherigen Ausführungsbeispiels gemäß den Figuren 7 bis 12 weist die weibliche Schnittstelleneinrichtung 9 gemäß diesen beiden Ausführungsbeispielen (Figuren 13 bis 15) keine zweite elektronische Schnittstelle 340 auf, da die chirurgischen Assistenzsysteme 202, 204 passiv ausgebildet sind. In dem entsprechenden Abschnitt der Stirnseite 368 ist lediglich eine einzige Ausnehmung 424 vorgesehen, um die Blöcke 341, 342, 343, 344 der männlichen Schnittstelleneinrichtung 8 aufnehmen zu können.

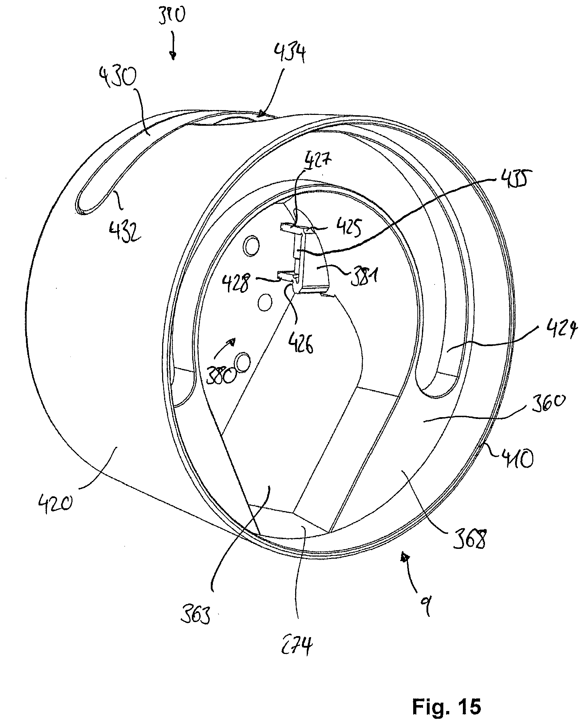

Ein weiterer Unterschied zum chirurgischen Assistenzsystem 200 besteht darin, dass die ersten und zweiten Bolzen 381, 382 (in Fig. 15 nur einer zu sehen) der mechanischen Kopplungsmitteln 380 ebenfalls aus Kunststoff ausgebildet sind und mittels Filmscharnieren 425, 426 mit entsprechenden Stegen 427, 428 verbunden sind, die ihrerseits an dem Grundkörper 420 angelenkt sind. Über die Filmscharniere 425, 426 sind die ersten und zweiten Bolzen 381, 382 auch in die Arretierposition vorgespannt. Es ist also ein Festkörpergelenk mit Festkörpervorspannung bereitgestellt.

Zum Verbringen der mechanischen Kopplungsmittel 380 gemäß diesen zweiten und dritten Ausführungsbeispielen (Figuren 13 bis 15) in die Freigabeposition, bzw. Freigabestellung, ist ein Hebel 430 vorgesehen, der an einer Ausnehmung 432 der Umfangsfläche des Grundkörpers 420 im Wesentlichen flächenbündig aufgenommen ist. Der Hebel 430 ist über eine Fingermulde 434 manuell betätigbar. Durch Anheben des Hebels 430 wird über einen Stab 435 der Bolzen 381 angehoben 8 (das Gleiche gilt auch für den zweiten Bolzen 382) und das chirurgische Assistenzsystem 202, 204 kann auf die männliche Schnittstelleneinrichtung 8 aufgesteckt werden.

Zwischen dem Hebel 430 und einem darunterliegenden Abschnitt des Grundkörpers 420 ist vorzugsweise eine Rastverbindung vorgesehen, sodass der Hebel 430 an dem Grundkörper 420 einrasten kann, um die Kopplung zwischen der weiblichen Schnittstelleneinrichtung 9 des chirurgischen Assistenzsystems 202, 204 und der männlichen Schnittstelleneinrichtung 8 zu fixieren.

Die chirurgischen Assistenzsysteme 202, 204 (Figuren 13 bis 15) können vollständig als Single-use-Produkte hergestellt werden. Anstelle der Stirnseiten 412 können auch direkt Ankopplungsmechanismen zum Ankoppeln von Instrumenten an den Grundkörper 420 geschraubt werden, oder einstückig mit diesem verbunden werden. Der Körper 420 ist vorzugsweise aus einem isolierenden Kunststoff gebildet, sodass im Falle einer passiven Verwendung eines Instruments das chirurgische Assistenzsystem 202, 204 gegenüber der männlichen Schnittstelleneinrichtung 8 und etwa dem Haltearm 1 eine elektrische Isolierung bildet. An das axiale Ende des Kragens 410 kann insbesondere eine sterile Hülle eingeschweißt sein, oder sterile Folie eingeschweißt sein, die über einen Teil des Haltearms 1 gezogen werden kann. Auch ist es denkbar, dass das chirurgische Assistenzsystem 202, 204 insgesamt mit einer sterilen Hülle verpackt bereitgestellt wird. Auf diese Weise kann ein Operateur direkt vor Inbetriebnahme das chirurgische Assistenzsystem 202, 204 auspacken und dieses bleibt möglichst lange steril.

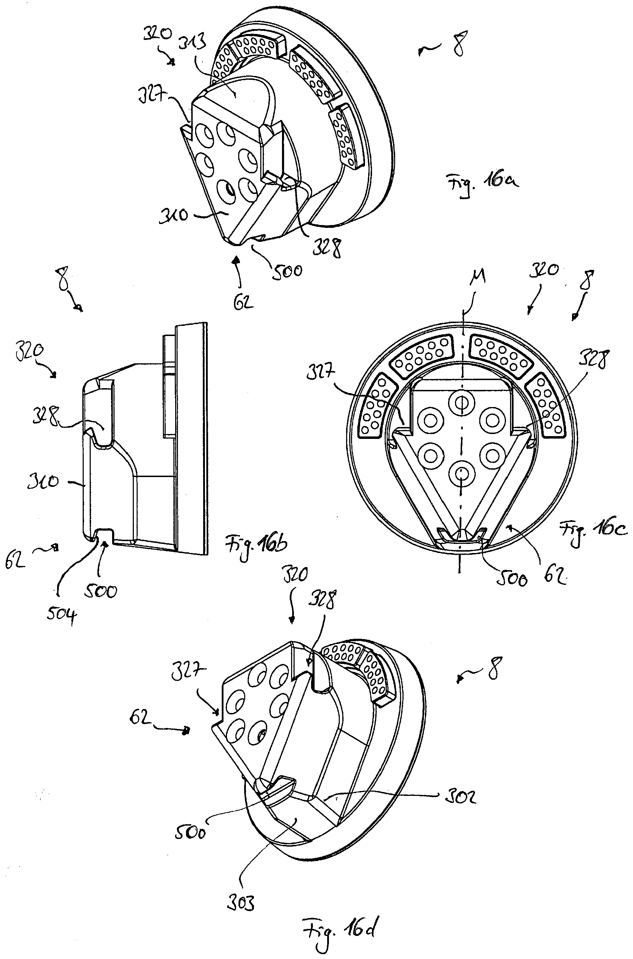

Die Figuren 16a bis 18 zeigen ein zweites Ausführungsbeispiel der männlichen Schnittstelleneinrichtung 8 und der korrespondierenden weiblichen Schnittstelleneinrichtung 9. Gleiche und ähnliche Elemente sind mit denselben Bezugszeichen wie in den vorherigen Ausführungsbeispielen bezeichnet, sodass im Folgenden vollumfänglich auf die obige Beschreibung Bezug genommen wird. Im Folgenden werden in erster Linie die Unterschiede zu den vorherigen Ausführungsformen beschrieben.

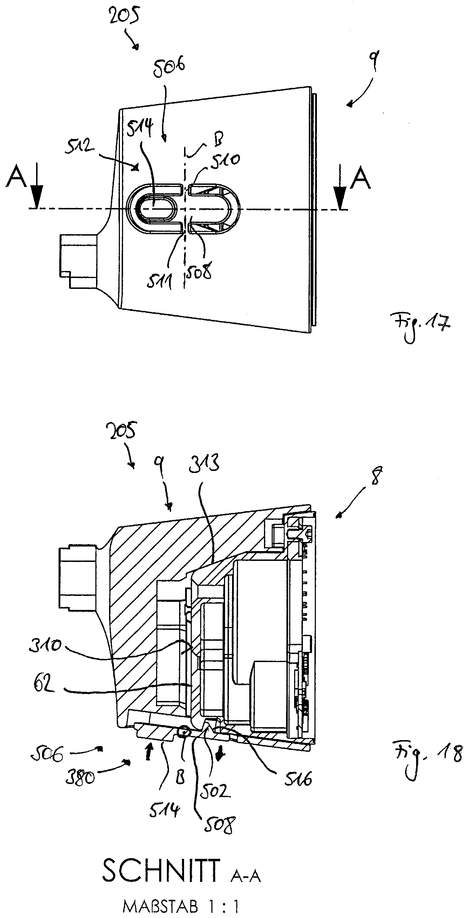

Wie insbesondere in den Figuren 16a-16d zu erkennen ist, weisen die ersten mechanischen Kopplungsmittel 320 eine dritte Hinterschneidung 500 auf. Die dritte Hinterschneidung wirkt mit einem dritten Bolzen 502 (vgl. Fig. 18) zusammen, der in diese eingreifen kann. Bezogen auf eine Mittelebene M (vgl. Fig. 16c) ist die dritte Hinterschneidung 500 im Wesentlichen zwischen der ersten Hinterschneidung 327 und der zweiten Hinterschneidung 328 angeordnet. Hierdurch kann eine bessere Symmetrie bei der Kräfteverteilung erreicht werden. Zur axialen Stirnseite 310 hin weist die dritte Hinterschneidung 500 eine dritte Flanke 504 auf, die schräg bezogen auf die Zentralachse L, aber im Wesentlichen senkrecht zur Nasenfläche 303, der Nase 302 ausgerichtet ist. Die dritte Hinterschneidung 500 ist in den Figuren seitlich offen, kann aber ebenso seitlich geschlossen sein.

In Richtung der Mittelebene M ist die dritte Hinterschneidung 500 mit Bezug auf Figur 16c nach unten offen, während die ersten und zweiten Hinterschneidungen 327, 328 nach oben offen sind. Die ersten und zweiten Bolzen 381, 382 greifen entgegengesetzt zum dritten Bolzen 502 in die entsprechenden Hinterschneidungen ein.

Figur 18 zeigt nun die männliche Schnittstelleneinrichtung 8 gemäß den Figuren 16a bis 16d in einer in einem chirurgischen Assistenzsystem 205 ausgebildeten weiblichen Schnittstelleneinrichtung 9 gemäß dem zweiten Ausführungsbeispiel. Das chirurgische Assistenzsystem 205 ist passiv und hat keine Elektronik. Es dient beispielsweise als Adapter zur Aufnahme von weiteren medizinischen Instrumenten.

Die weibliche Schnittstelleneinrichtung 9 weist den dritten Bolzen 502 auf, der in diesem Fall schwenkbar um eine Bolzenschwenkachse B angeordnet ist. Die zweiten mechanischen Kopplungsmittel 380 der weiblichen Schnittstelleneinrichtung 9 weisen gemäß diesem Ausführungsbeispiel nur den dritten Bolzen 502 auf, und keine ersten und/oder zweiten Bolzen 381, 382. Die ersten und zweiten Hinterschneidungen 327, 328 sind in diesem Fall funktionslos.

Die weibliche Schnittstelleneinrichtung 9 weist demnach auch eine zweite Betätigungseinrichtung 506 auf, die dazu vorgesehen ist, den dritten Bolzen 502 in einer Freigabestellung zu verbringen. Die zweite Betätigungseinrichtung 506 umfasst in diesem Ausführungsbeispiel eine Wippe 508, die über zwei Filmscharniere 510, 511 mit dem Körper 512 des chirurgischen Assistenzsystems 205 verbunden ist. Die Wippe 508 hat einen Tastbereich 514, der beispielsweise mit dem Daumen drückbar ist. Wird dieser in Richtung des Pfeils (vgl. Fig. 18) gedrückt, rotiert die Wippe 508 um die Bolzenschwenkachse B und der dritte Bolzen 502 bewegt sich in Richtung des entsprechenden Pfeils in Figur 18; bei entsprechend weiter Rotation kommt er aus der dritten Hinterschneidung 500 heraus, und gibt diese frei.

Die Filmscharniere 510, 511 wirken hier wie Federn, sodass der dritte Bolzen 502 in die Arretierstellung federbelastet vorgespannt ist.

Der dritte Bolzen 502 ist, wie dies auch schon mit Bezug auf den ersten und zweiten Bolzen 381, 382 beschrieben wurde, angeschrägt, und weist eine schräge Flanke 516 auf. Hierdurch kann die weibliche Schnittstelleneinrichtung 9 einfach auf die männliche Schnittstelleneinrichtung 8 aufgesteckt werden, wobei der dritte Bolzen 502 durch die schräge Flanke 516 bei Kontakt mit der Nase 302 ausgelenkt wird, und bei weiterem Aufschieben der weiblichen Schnittstelleneinrichtung 9 in die dritte Hinterschneidung 500 selbsttätig einrastet.

In anderen Ausführungsformen kann auch vorgesehen sein, dass der dritte Bolzen 502 durch die (erste) Betätigungseinrichtung 380 betätigbar ist. Auch ist ein Rotieren des dritten Bolzens 502 nicht zwingend, und die Erfindung ist nicht hierauf beschränkt. Es sind auch Ausführungsformen umfasst, bei denen der dritte Bolzen 502 verschieblich ist.

## Patentansprüche

1. Medizinische mechatronische männliche Schnittstelleneinrichtung (8) zum Koppeln eines chirurgischen Assistenzsystems (200, 202, 204) an eine Haltestruktur (1), mit
einer Basis (58), die eine Stirnseite (60) aufweist, wobei sich von der Stirnseite (60) ein Funktionskörper (62) erstreckt,
ersten mechanischen Kopplungsmitteln (320) zum form- und/oder kraftschlüssigen Koppeln mit einer korrespondierenden medizinischen mechatronischen weiblichen Schnittstelleneinrichtung (9), sowie wenigstens einer ersten elektronischen Schnittstelle (340) zum Verbinden mit einer korrespondierenden zweiten elektronischen Schnittstelle (292) der weiblichen Schnittstelleneinrichtung (9),
wobei der Funktionskörper (62) eine Zentriereinrichtung (301) zum spielfreien Zentrieren des chirurgischen Assistenzsystems (200, 202, 204, 205) gegenüber der Basis (58) aufweist, **dadurch gekennzeichnet, dass** die Zentriereinrichtung (301) eine erste Funktionsfläche (311), eine zweite Funktionsfläche (312) und eine dritte Funktionsfläche (313) umfasst, die nicht-parallel zueinander sind und deren Normalen nicht in einer gemeinsamen Ebene liegen, wobei die Funktionsflächen (311, 312, 313) gemeinsam wenigstens teilweise eine dreiseitige Pyramide definieren.

2. Männliche Schnittstelleneinrichtung nach Anspruch 1, wobei der Funktionskörper (62) spiegelsymmetrisch ist.

3. Männliche Schnittstelleneinrichtung nach einem der vorstehenden Ansprüche, wobei die Funktionsflächen (311, 312, 313) jeweils wenigstens 20° zu einer Zentralachse (L) des Funktionskörpers (62) geneigt sind.

4. Männliche Schnittstelleneinrichtung nach einem der vorstehenden Ansprüche, wobei die ersten mechanischen Kopplungsmittel (320) wenigstens eine erste Hinterschneidung (327) an dem Funktionskörper (62) aufweisen, und vorzugsweise eine zweite Hinterschneidung (328) an dem Funktionskörper (62) aufweisen, die spiegelsymmetrisch zur ersten Hinterschneidung (327) ausgebildet ist.

5. Männliche Schnittstelleneinrichtung nach einem der vorstehenden Ansprüche, wobei die ersten mechanischen Kopplungsmittel (320) eine dritte Hinterschneidung (500) an dem Funktionskörper (62) umfassen, wobei die dritte Hinterschneidung (500) im Wesentlichen entgegengesetzt zu der ersten und/oder zweiten Hinterschneidung (327, 328) ausgebildet ist, sodass ein entsprechender Vorsprung in einer mit Bezug auf die erste und/oder zweite Hinterschneidung (327, 328) entgegengesetzte Richtung in die dritte Hinterschneidung (500) eingreifen kann.

6. Männliche Schnittstelleneinrichtung nach einem der vorstehenden Ansprüche, wobei die Funktionsflächen (311, 312, 313) radial in eine Umfangsfläche (304) eines Grundkörpers (300) des Funktionskörpers (62) auslaufen, wobei der Grundkörper (300) vorzugsweise nicht rotationssymmetrisch ist, und vorzugsweise zylindrisch mit einer radialen Nase (302) ausgebildet ist.

7. Männliche Schnittstelleneinrichtung nach einem der vorstehenden Ansprüche, wobei die erste elektronische Schnittstelle (340) an der Stirnseite (60) der Basis (58) ausgebildet ist.

8. Männliche Schnittstelleneinrichtung nach Anspruch 7, wobei die erste elektronische Schnittstelle (340) wenigstens zwei Erdungskontakte (345, 346) aufweist, die derart angeordnet sind, dass wenigstens einer von diesen bei Kopplung mit einer korrespondierenden weiblichen Schnittstelleneinrichtung (9) zuerst mit wenigstens einem korrespondierenden Erdungskontakt der weiblichen Schnittstelleneinrichtung (9) in Kontakt kommt, bevor weitere elektrische Kontakte geschlossen werden.

9. Medizinische weibliche Schnittstelleneinrichtung (9) zum Koppeln eines chirurgischen Assistenzsystems (200) an eine Haltestruktur, wobei die weibliche Schnittstelleneinrichtung (9) zum Koppeln mit der männlichen Schnittstelleneinrichtung (8) nach einem der vorstehenden Ansprüche ausgebildet ist, mit:
einem Rahmen (360), der eine zentrale Ausnehmung (274) zur Aufnahme des Funktionskörpers (62) definiert,
zweiten mechanischen Kopplungsmitteln (380) zum form- und/oder kraftschlüssigen Koppeln mit den ersten mechanischen Kopplungsmitteln (320) der männlichen Schnittstelleneinrichtung (8),
wobei die zentrale Ausnehmung (274) eine Zentriereinrichtung (362) zum spielfreien Zentrieren des chirurgischen Assistenzsystems (200, 202, 204) gegenüber der Basis (58) der ersten Schnittstelleneinrichtung (8) aufweist, wobei die Zentriereinrichtung (362) eine erste Funktionsfläche (363), eine zweite Funktionsfläche (364) und eine dritte Funktionsfläche (365) umfasst, die mit den ersten, zweiten und dritten Funktionsflächen (311, 312, 313) der männlichen Schnittstelleneinrichtung (8) zum flächigen Kontaktieren korrespondieren.

10. Weibliche Schnittstelleneinrichtung (9) nach Anspruch 9, die mechatronisch ausgebildet ist und wenigstens eine zweite elektronische Schnittstelle (370) zum Verbinden mit der ersten elektronischen Schnittstelle (320) der männlichen Schnittstelleneinrichtung (8) aufweist.

11. Weibliche Schnittstelleneinrichtung (9) nach Anspruch 9 oder 10, wobei die zweiten mechanischen Kopplungsmittel (380) wenigsten einen ersten beweglichen Bolzen (381) zum Hintergreifen der ersten Hinterschneidung (327) der ersten mechanischen Kopplungsmittel (320) aufweisen, wobei der ersten Bolzen (381) vorzugsweise in eine Arretierstellung vorgespannt, und vorzugsweise an einer freien Spitze angeschrägt (383) ist.

12. Weibliche Schnittstelleneinrichtung (9) nach einem der Ansprüche 9 bis 11, aufweisend eine Betätigungseinrichtung (390) zum Versetzen der zweiten mechanischen Kopplungsmittel (380) in eine Freigabestellung.

13. Weibliche Schnittstelleneinrichtung (9) nach Anspruch 11 und 12, wobei die Betätigungseinrichtung (390) einen schwenkbar am Rahmen (360) gelagerten Hebel (392) mit einem Griffabschnitt (394) aufweist, der abtriebsseitig mit dem ersten Bolzen (381) in Kontakt steht, wobei durch Betätigung des Griffabschnitts (392) der erste Bolzen (381) von der Arretierstellung in eine Freigabestellung bringbar ist.

14. Haltearm (1) für medizinische Zwecke zum Halten eines chirurgischen Assistenzsystems (200, 202, 204) nach Anspruch 1, mit
einem proximalen Ende (2) zum Befestigen des Haltearms (1) an einer Basis und einem distalen Ende (4) zum Aufnehmen des chirurgischen Assistenzsystems (200);
zwei oder mehr Armsegmenten (10, 12, 14, 16, 18, 20, 22) und zwei oder mehr Gelenken (11, 13, 15, 17, 19, 21, 23), mittels denen die Armsegmente (10, 12, 14, 16, 18, 20, 22) gelenkig miteinander verbunden sind;
einer ersten Schnittstelle (6) an dem proximalen Ende (2) zum Verbinden des Haltearms (1) mit einer Energiequelle sowie mit einer externen Steuereinheit zum Übertragen von Signalen an den und von dem Haltearm (1);
einer zweiten Schnittstelle (8) an dem distalen Ende (4) zum Koppeln des Haltearms (1) mit dem Assistenzsystem (200, 202, 204) zum Steuern des Assistenzsystems (200, 202, 204);
und einer Übertragungseinrichtung, welche innerhalb des Haltearms (1) angeordnet ist und die erste Schnittstelle (6) mit der zweiten Schnittstelle (8) zum Übertragen von Energie und Signalen zwischen den Schnittstellen (6, 8) verbindet,
wobei die zweite Schnittstelle (8) eine medizinische mechatronische männliche Schnittstelleneinrichtung (8) nach einem der vorstehenden Ansprüche 1 bis 8 aufweist.

15. Chirurgisches Assistenzsystem (200, 202, 204) nach Anspruch 1 mit einer medizinischen weiblichen Schnittstelleneinrichtung (9) nach einem der Ansprüche 9 bis 13.

16. Passive Adaptervorrichtung (202, 204) mit einem napfförmigen Grundkörper (420) und einer medizinischen weiblichen Schnittstelleneinrichtung (9) nach einem der Ansprüche 9 bis 13.

## Claims

1. A medical mechatronic male interface device (8) for coupling a surgical assistance system (200, 202, 204) to a support structure (1), having
a base (58) comprising an end face (60), a functional body (62) extending from the end face (60),
first mechanical coupling means (320) for positive and/or non-positive coupling to a corresponding medical mechatronic female interface device (9), and at least one first electronic interface (340) for connecting to a corresponding second electronic interface (292) of the female interface device (9),
the functional body (62) comprising a centring device (301) for centring the surgical assistance system (200, 202, 204, 205) free of play relative to the base (58), **characterized in that**
the centring device (301) comprises a first functional surface (311), a second functional surface (312) and a third functional surface (313) which are non-parallel to each other and the normals of which do not lie in a common plane, wherein the functional surfaces (311, 312, 313) together at least partially define a three-sided pyramid.

2. The male interface device according to claim 1, wherein the functional body (62) is mirror-symmetrical.

3. The male interface device according to any of the preceding claims, wherein the functional surfaces (311, 312, 313) are each inclined at least 20° to a central axis (L) of the functional body (62).

4. The male interface device according to any of the preceding claims, wherein the first mechanical coupling means (320) comprise at least a first undercut (327) on the functional body (62), and preferably comprise a second undercut (328) on the functional body (62), which is formed mirror-symmetrical to the first undercut (327).

5. The male interface device according to any of the preceding claims, wherein the first mechanical coupling means (320) include a third undercut (500) on the functional body (62), wherein the third undercut (500) is formed substantially opposite to the first and/or second undercuts (327, 328) such that a protrusion in an opposite direction with regard to the first and/or second undercut (327, 328) can engage the third undercut (500).

6. The male interface device according to any of the preceding claims, wherein the functional faces (311, 312, 313) radially taper off into a circumferential surface (304) of a base body (300) of the functional body (62), wherein the base body (300) is preferably not rotationally symmetrical, and is preferably formed cylindrical with a radial nose (302).

7. The male interface device according to any of the preceding claims, wherein the first electronic interface (340) is formed on the end face (60) of the base (58).

8. The male interface device according to claim 7, wherein the first electronic interface (340) comprises at least two ground contacts (345,346) arranged such that during coupling to a corresponding female interface device (9) at least one of these first contacts a corresponding ground contact of the female interface device (9) before further electrical contacts are closed.

9. A medical female interface device (9) for coupling a surgical assistance system (200) to a support, wherein the female interface device (9) is adapted to couple to a male interface device (8) according to any of the preceding claims, having:
a frame (360) defining a central recess (274) for receiving the functional body (62),
second mechanical coupling means (380) for positive and/or non-positive coupling to the first mechanical coupling means (320) of the male interface device (8),
wherein the central recess (274) comprising a centring device (362) for centring the surgical assistance system (200, 202, 204) free of play relative to the base (58) of the first interface device (8), wherein the centring device (362) comprises a first functional face (363), a second functional face (364) and a third functional face (365) corresponding to first, second and third functional faces (311, 312, 313) of the male interface device (8) for areal contacting.

10. The female interface device (9) according to claim 9, which is mechatronically implemented and comprises at least one second electronic interface (370) for connecting to the first electronic interface (320) of the male interface device (8).

11. The female interface device (9) according to claim 9 or 10, wherein the second mechanical coupling means (380) comprise at least a first movable bolt (381) for engaging behind the first undercut (327) of the first mechanical coupling means (320), wherein the first bolt (381) is preferably biased to a locking position and is preferably tapered at a free tip (383).

12. The female interface device (9) according to any of the claims 9 to 11, comprising an actuation device (390) for offsetting the second mechanical coupling means (380) into a release position.

13. The female interface device (9) according to claims 11 and 12, wherein the actuation device (390) comprises a lever (392) pivotally supported on the frame (360) having a handle portion (394) which is connected to the first bolt (381) on the output side, wherein the bolt (381) can be brought from the locking position to a release position by actuating the handle portion (392).

14. A support arm (1) for medical purposes for supporting a surgical assistance system (200, 202, 204) according to claim 1, having
a proximal end (2) for fixing the support arm (1) to a base and a distal end (4) for receiving a surgical assistance system (200);
two or more arm segments (10, 12, 14, 16, 18, 20, 22) and two or more joints (11, 13, 15, 17, 19, 21, 23) by means of which the arm segments (10, 12, 14, 16, 18, 20, 22) are connected to each other in an articulated manner;
a first interface (6) on the proximal end (2) for connecting the support arm (1) to a power source and to an external control unit for transmitting signals to and from the support arm (1);
a second interface (8) on the distal end (4) for coupling the support arm (1) to the assistance system (200, 202, 204) for controlling the assistance system (200, 202, 204);
and a transmission device, arranged inside the support arm (1) connecting the first interface (6) to the second interface (8) for transmitting power and signals between the interfaces (6,8),
wherein the second interface (8) comprises a medical mechatronic male interface device (8) according to any of the preceding claims 1 to 8.

15. A surgical assistance system (200, 202, 204) according to claim 1 having a medical female interface device (9) according to any of the claims 9 to 13.

16. A passive adapter device (202, 204) having a cup-shaped base body (420) and a medical female interface device (9) according to any of the claims 9 to 13.

## Revendications

1. Dispositif (8) médical mécatronique d'interface mâle de couplage d'un système (200, 202, 204) d'assistance chirurgicale à une structure (1) de maintien, comprenant
une embase (58), qui a une face (60) frontale, une pièce (62) fonctionnelle s'étendant de la face (60) frontale,
des premiers moyens (320) d'accouplement mécaniques pour l'accouplement à complémentarité de forme et/ou de force à un dispositif (9) médical mécatronique d'interface femelle correspondant, ainsi qu'au moins une première interface (340) électronique de liaison à une deuxième interface (292) électronique correspondante du dispositif (9) d'interface femelle,
dans lequel la pièce (62) fonctionnelle a un dispositif (301) de centrage pour le centrage sans jeu du système (200, 202, 204, 205) d'assistance chirurgical par rapport à l'embase (58),
**caractérisé en ce que** le dispositif (301) de centrage comprend une première surface (311) fonctionnelle, une deuxième surface (312) fonctionnelle et une troisième surface (313) fonctionnelle, qui ne sont pas parallèles entre elles et dont les normales ne sont pas dans un plan commun, les surfaces (311, 312, 313) fonctionnelles définissant conjointement, au moins en partie, une pyramide triangulaire.

2. Dispositif d'interface mâle suivant la revendication 1, dans lequel la pièce (62) fonctionnelle est de symétrie comme en un miroir.

3. Dispositif d'interface mâle suivant l'une des revendications précédentes, dans lequel les surfaces (311, 312, 313) fonctionnelles sont inclinées chacune d'au moins 20° par rapport à un axe (L) central de la pièce (62) fonctionnelle.

4. Dispositif d'interface mâle suivant l'une des revendications précédentes, dans lequel les premiers moyens (320) d'accouplement mécaniques ont au moins une première contre-dépouille (327) sur la pièce (62) fonctionnelle et, de préférence, une deuxième contre-dépouille (328) sur la pièce (62) fonctionnelle, qui est symétrique comme en un miroir de la première contre-dépouille (327).

5. Dispositif d'interface mâle suivant l'une des revendications précédentes, dans lequel les premiers moyens (320) d'accouplement mécaniques comprennent une troisième contre-dépouille (500) sur la pièce (62) fonctionnelle, la troisième contre-dépouille (500) étant constituée de manière sensiblement opposée à la première et/ou à la deuxième contre-dépouille (327, 328), de manière à ce qu'une saillie correspondante puisse pénétrer dans la troisième contre-dépouille (500) dans un sens opposé par rapport à la première et/ou à la deuxième contre-dépouille (327, 328).

6. Dispositif d'interface mâle suivant l'une des revendications précédentes, dans lequel les surfaces (311, 312, 313) fonctionnelles aboutissent radialement dans une surface (304) de pourtour d'une pièce (300) de base de la pièce (62) fonctionnelle, la pièce (300) de base n'étant, de préférence, pas de révolution et étant constituée, de préférence, de manière cylindrique en ayant un bec (302) radial.

7. Dispositif d'interface mâle suivant l'une des revendications précédentes, dans lequel la première interface (340) électronique est constituée sur la face (60) frontale de l'embase (58).

8. Dispositif d'interface mâle suivant la revendication 7, dans lequel la première interface (340) électronique a au moins deux contacts (345, 346) de mise à la terre, qui sont disposés de manière à ce qu'au moins l'un d'entre eux, lors de l'accouplement à un dispositif (9) d'interface femelle correspondant, vienne en contact d'abord avec au moins un contact de mise à la terre correspondant du dispositif (9) d'interface femelle, avant que d'autres contacts électriques ne soient fermés.

9. Dispositif (9) médical d'interface femelle pour l'accouplement d'un système (20) d'assistance chirurgicale à une structure de maintien, le dispositif (9) d'interface femelle étant constitué pour l'accouplement au dispositif (8) d'interface mâle suivant l'une des revendications précédentes, comprenant :
un cadre (360), qui définit un évidement (274) central de réception de la pièce (62) fonctionnelle,
des deuxièmes moyens (380) d'accouplement mécaniques pour l'accouplement à complémentarité de forme et/ou de force aux premiers moyens (320) d'accouplement mécaniques du dispositif (8) d'interface mâle,
dans lequel l'évidement (274) central a un dispositif (362) de centrage pour le centrage sans jeu du système (200, 202, 204) d'assistance chirurgicale par rapport à l'embase (58) du premier dispositif (8) d'interface, le dispositif (362) de centrage comprenant une première surface (363) fonctionnelle, une deuxième surface (364) fonctionnelle et une troisième surface (365) fonctionnelle, qui correspondent, pour la mise en contact en nappe, aux première, deuxième et troisième surfaces (3141, 312, 313) fonctionnelles du dispositif (8) d'interface mâle.

10. Dispositif (9) médical d'interface femelle suivant la revendication 9, qui est constitué mécanotriquement et qui a au moins une deuxième interface (370) électronique de liaison à la première interface (320) électronique du dispositif (8) d'interface mâle.

11. Dispositif (9) médical d'interface femelle suivant la revendication 9 ou 10, dans lequel les deuxièmes moyens (380) d'accouplement mécaniques ont au moins une première cheville (380) mobile pour prendre par derrière la première contre-dépouille (327) des premiers moyens (320) d'accouplement mécaniques, la première cheville (381) étant, de préférence, précontrainte dans une position d'arrêt et étant, de préférence, biseautée (383) à une pointe libre.

12. Dispositif (9) médical d'interface femelle suivant l'une des revendications 9 à 11, comportant un dispositif (390) d'actionnement pour mettre les deuxièmes moyens (380) d'accouplement mécaniques dans une position de libération.

13. Dispositif (9) médical d'interface femelle suivant la revendication 11 et 12, dans lequel le dispositif (390) d'actionnement a un levier (392), monté pivotant sur le cadre (360) et ayant une partie (394) de préhension, qui est en contact, du côté de la sortie, avec la première cheville (381), dans lequel, par actionnement de la partie (392) de préhension, la première cheville (381) peut être mise de la position d'arrêt à une position de libération.

14. Bras (1) de maintien à des fins médicales pour le maintien d'un système (200, 202, 204) d'assistance chirurgicale suivant la revendication 1, comprenant
une extrémité (2) proximale pour la fixation du bras (1) de maintien à une embase et une extrémité (4) distale pour la réception du système (200) d'assistance chirurgicale ;
deux ou plusieurs segments (10, 12, 14, 16, 18, 20, 22) de bras et deux ou plusieurs articulations (11, 13, 15, 17, 17, 19, 21, 23), au moyen desquelles les segments (10, 12, 014, 16, 18, 20, 22) de bras sont articulés entre eux ;
une première interface (6) à l'extrémité (2) proximale pour la liaison du bras (1) de maintien à une source d'énergie, ainsi qu'à une unité extérieure de commande pour la transmission de signaux au bras (1) de maintien et du bras (1) de maintien ;
une deuxième interface (8) à l'extrémité (4) distale pour l'accouplement du bras (1) de maintien au système (200, 202, 204) d'assistance, afin de commander le système (200, 2020, 204) d'assistance ;
et un dispositif de transmission, qui est disposé à l'intérieur du bras (1) de maintien et qui relie la première interface (6) à la deuxième interface (8) pour la transmission d'énergie et de signaux entre les interfaces (6, 8),
dans lequel la deuxième interface (8) a un dispositif (8) médical mécatronique d'interface mâle suivant l'une des revendications précédentes 1 à 8.

15. Système (200, 202, 204) d'assistance chirurgicale suivant la revendication 1, comprenant un dispositif médical mécatronique d'interface femelle suivant l'une des revendications 9 à 13.

16. Installation (202, 204) passive d'adaptateur ayant une pièce (420) de base en forme de godet et un dispositif (9) médical d'interface femelle suivant l'une des revendications 9 à 13.
